(19)

Europäisches
Patentamt
European
Patent Office
Office européen
des brevets

(11)   **EP 3 699 922 A1**

(12)                **EUROPÄISCHE PATENTANMELDUNG**

(43) Veröffentlichungstag:
**26.08.2020 Patentblatt 2020/35**

(21) Anmeldenummer: **19158311.1**

(22) Anmeldetag: **20.02.2019**

(51) Int Cl.:
**G16H 40/40** (2018.01)          **G16H 40/60** (2018.01)
**G06F 21/00** (2013.01)           **H04L 29/08** (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder:
• **Fresenius Medical Care Deutschland GmbH
61352 Bad Homburg (DE)**
• **Fresenius Medical Care AG & Co. KGaA
61352 Bad Homburg (DE)**

(72) Erfinder:
• **Hellhund, Jonas
60438 Frankfurt (DE)**
• **Lindemann, Robert
65193 Wiesbaden (DE)**
• **Peters, Arne
61352 Bad Homburg (DE)**
• **Fischer, Gerome
99947 Weberstedt (DE)**

(74) Vertreter: **Schwarz, Claudia et al
Schwarz + Kollegen
Patentanwälte
Hermann-Schmid-Straße 10
80336 München (DE)**

(54)     **MANIPULATIONSSICHERE DOKUMENTATION VON EINER VERWENDUNG VON
VERBRAUCHSGEGENSTAENDEN BEIM BETRIEB EINES DIALYSEGERAETES**

(57)     Die vorliegende Erfindung betrifft ein Dokumentationsmodul (DM) und ein Dialysegerät (DG) oder eine Umkehrosmoseanlage (RO) mit einer solchen Verarbeitungseinheit, ein Verfahren sowie ein System zur Dokumentation eines Gerätebetriebs im medizinischen Umfeld vor einem nicht autorisierten Betrieb des Gerätes, wobei die Autorisierung abhängig ist von einem Verknüpfungsereignis zwischen zwei Verknüpfungspartnern im Rahmen eines Gerätebetriebs, wobei alle oder ausgewählte Geräte (DG, RO) über ein Netzwerk datentechnisch verbunden sind und wobei ein erster Verknüpfungspartner ein Gebrauchsgegenstand ist, der bei dem Gerätebetrieb verwendet wird. Dabei umfasst das System mehrere Geräte (DG, RO) eines Geräteverbundes, wobei jeweils ein Gerät (DG, RO) ein Dokumentationsmodul enthält, umfassend:
- eine Einleseschnittstelle (I) zum Einlesen eines ersten Identifikators (ID-P, ID-RO) und eines zweiten Identifikators (ID-D);
- eine elektronische Verarbeitungseinheit (V), die ausgebildet ist, ein Dokumentationsverfahren auszuführen;
- einen Speicher (MEM) zur Speicherung von einem ersten berechneten Wert (W1) und einem zweiten berechneten Wert (W2) in einer Distributed Ledger Struktur (DL);
- einer Schnittstelle zu dem Netzwerk, über das die Geräte in Datenaustausch stehen.

Fig. 1

**EP 3 699 922 A1**

**Beschreibung**

Die vorliegende Erfindung liegt auf dem Gebiet der Medizintechnik und betrifft den Betrieb von medizinischen Geräten, wie Dialysegeräten und Umkehrosmose-Anlagen, die mit Verbrauchsgegenständen (z.B. Einwegartikeln, Disposables) betrieben werden. Die Erfindung betrifft insbesondere ein Verfahren zur manipulationssicheren Dokumentation über die verwendeten Verbrauchsgegenstände beim Betrieb der Geräte sowie ein Computerprogramm, ein Dokumentationsmodul, ein medizinisches Gerät und ein System.

Der Betrieb von medizinischen Geräten weist insofern im Vergleich zu anderen nichtmedizinischen Betriebsverfahren eine Besonderheit auf, dass der Gerätebetrieb die Gesundheit eines Patienten betrifft. Insofern sind hier besondere Sicherungsmaßnahmen einzuhalten, insbesondere wenn die Verwendung von Verbrauchsgegenständen (Disposables, wie z.B. einem Dialysator) erforderlich ist, deren Verwendung limitiert ist. So erfolgt beispielsweise eine Dialysebehandlung bei einer Hämodialyse an einem Dialysegerät, das sich z.B. an einem Dialysezentrum befinden kann, üblicherweise derart, dass sich ein Patient an dem Dialysegerät registriert, um die Dialyse durchzuführen. Bei der Dialyse wird das Blut des Patienten durch einen extrakorporalen Kreislauf geführt, der z.B. als Einwegartikel ausgestaltet sein kann. Dazu kann ein vom Patienten mitgeführter oder für den Patienten bereitgestellter Satz von Verbrauchsgegenständen (z.B. Schlauchsatz mit Schlauchleitungen, Filtermodulen, Konnektoren, Tropfkammern etc.) verwendet werden.

Zur Behandlung selbst kann der Patient unterschiedliche Dialysegeräte verwenden, die sich in demselben oder auch in unterschiedlichen Dialysezentren (mitunter weltweit verteilt) befinden. Aus medizinischer Perspektive ist es wichtig, sicherzustellen, dass der Satz von Verbrauchsgegenständen auf vorgesehene Weise verwendet wird. Bestimmte Anwendungen erfordern es z.B., dass der Satz von Verbrauchsgegenständen nur einmal verwendet werden darf, während andere Anwendungen eine begrenzte Anzahl Wiederverwendungen zulassen. Auf jeden Fall ist jedoch sicherzustellen, dass der Satz von Verbrauchsgegenständen nur für genau einen Patienten verwendet wird (er darf somit nicht "ausgeliehen" werden an Mitpatienten). Eine Verwendung auf unterschiedlichen Maschinen kann jedoch erlaubt sein.

Stand der Technik

Bei bekannten Systemen aus dem Stand der Technik wurde die Autorisierung einer Verwendung von Verbrauchsgegenständen bisher nur unzureichend überprüft. In der Regel erfolgte dies durch das Behandlungspersonal manuell, z.B. durch manuelle Nachfrage bei dem Patienten. Es liegt auf der Hand, dass ein solches Verfahren mit teilweise gravierenden Nachteilen verbunden ist. Zum einen konnte eine Überprüfung vom Personal schlichtweg vergessen werden und somit unterbleiben, zum anderen sind die Informationsquellen nicht verlässlich. Dies beeinträchtigt die Sicherheit des Gerätebetriebs und kann sich nachteilig auf den gesamten Behandlungsprozess auswirken.

Aus der WO2015024647A2 ist es beispielsweise bekannt, den Gebrauchsgegenstand mit einem Sensor zu versehen, um Messwerte im Rahmen der Behandlung zu erfassen und verarbeiten zu können. Dabei kann es sich z.B. um einen Füllstandsensor eines Dialysatbehälters als Beispiel eines Gebrauchsgegenstandes oder um einen Temperatursensor zur Erfassung der Temperatur handeln.

Vergegenwärtigt man sich die praktische Situation bei einer Dialysebehandlung, so ist eine möglichst hohe Flexibilität für den Patienten gewünscht. Er soll mitunter die Möglichkeit haben, sich an einem beliebigen Dialysegerät eines beliebigen Dialysezentrums (z.B. weltweit verteilt) behandeln zu lassen, um dort die Behandlung mit einem von ihm mitgeführten Satz von Verbrauchsgegenständen durchzuführen. Die lokale Überprüfung der Verwendung der Verbrauchsgegenstände an einem spezifischen Gerät ist somit nicht ausreichend.

Zudem ist es wichtig, dass die Sicherheit der bezüglich des Patienten, der Verbrauchsgegenstände etc. gespeicherten Daten den Anforderungen an den Datenschutz und insbesondere dem Schutz von PHI-Daten (private or protected health information) genügt und die gespeicherten Daten nicht von unbefugten Personen einsehbar sind.

Der vorliegenden Erfindung liegt deshalb die Aufgabe zugrunde, eine technische Umsetzung anzugeben, mittels der die Sicherheit eines Gerätebetriebs erhöht und das Risiko für den Patienten gesenkt werden kann und dabei die Flexibilität für den Betreiber oder für den Patienten z.B. durch Auswahl eines beliebigen Behandlungsgerätes zu gewährleisten oder noch zu erhöhen. Ferner soll die Nachvollziehbarkeit eines Gerätebetriebs, insbesondere von ausgeführten Behandlungen, verbessert werden, um Rückschlüsse auf die jeweiligen Geräte zu ermöglichen und deren Dokumentation zu verbessern. Des Weiteren soll die technische Umsetzung effizient sein, so dass lange Wartezeiten aufgrund von Überprüfungsmaßnahmen am Gerät vermieden werden können.

Diese Aufgabe wird durch die Gegenstände der unabhängigen Ansprüche gelöst, insbesondere durch ein Verfahren, ein Computerprogramm, ein Dokumentationsmodul, ein medizinisches Gerät mit einem solchen Dokumentationsmodul und durch ein System. Vorteilhafte Ausführungsformen der Erfindung sind in den abhängigen Ansprüchen und der nachfolgenden Beschreibung beschrieben.

Gemäß einem ersten Aspekt bezieht sich die Erfindung somit auf ein Verfahren zur manipulationssicheren Dokumentation eines Gerätebetriebs eines Gerätes aus einer Menge von Geräten. Das Gerät kann im medizinischen Umfeld z.B. im Bereich der Dialyse betrieben werden. Das Gerät wird mit zumindest zwei Verknüpfungspartnern betrieben, wobei die Menge von Geräten über ein Netzwerk verbunden sind. Das Verfahren umfasst folgende Verfahrensschritte:

- Einlesen eines ersten Identifikators zur eindeutigen Identifikation eines ersten Verknüpfungspartners und Einlesen eines zweiten Identifikators zur eindeutigen Identifikation eines zweiten Verknüpfungspartners, der ein Gebrauchsgegenstand zur Verwendung an oder mit dem Gerät ist;
- Anwenden zumindest einer ersten (vorzugsweise kryptologischen) Rechenvorschrift auf den eingelesenen ersten Identifikator zur Berechnung eines ersten Wertes und
- Anwenden zumindest einer zweiten (vorzugsweise kryptologischen) Rechenvorschrift auf den eingelesenen zweiten Identifikator zur Berechnung eines zweiten Wertes;
- Schreiben des berechneten ersten Wertes in einen ersten Bereich eines Datenblocks (wobei der Datenblock eine spezielle vordefinierte, feste Datenstruktur hat); das Schreiben wird vorzugsweise lokal auf dem jeweiligen Gerät oder einem ihm zugeordneten Gerät ausgeführt;
- Schreiben des berechneten zweiten Wertes in einen zweiten Bereich des (desselben) Datenblocks;
- Veranlassen einer synchronisierten Speicherung des zweiteilig beschriebenen Datenblocks, der ein Verknüpfungsereignis des ersten und zweiten Verknüpfungspartners repräsentiert, in Speichern von Geräten des Netzwerkes. Die synchronisierte Speicherung ist vorzugsweise dezentral und erfolgt in Speichern einer verteilten Datenstruktur, die vorzugsweise als distributed ledger ausgebildet sein kann.

Das vorgeschlagene Verfahren hat den Vorteil, dass die Sicherheit bei der Speicherung von Dokumentationsdaten über die Verwendung des Gebrauchsgegenstandes beim Gerätebetrieb erhöht werden kann. Die erfassten Dokumentationsdaten werden nur in verschlüsselter Form gespeichert und sind bei einem Angriff nicht im Klartext lesbar. Zudem kann durch die verteilte und redundante Speicherung der Sicherheitsgrad erhöht werden. Selbst wenn ein Angreifer einen Identifikator unberechtigterweise abfangen würde, so könnte er ihn nicht auslesen und zum Missbrauch - z.B. zur Kennzeichnung von gefälschten Produkten - verwenden. Die Dokumentationsdaten umfassen zumindest zwei Kategorien von Daten: Daten über oder zu dem ersten Verknüpfungspartner (z.B. erster Identifikator) und Daten über oder zu dem zweiten Verknüpfungspartner (z.B. zweiter Identifikator). Die Dokumentationsdaten können noch weitere Daten umfassen, insbesondere Daten zur technischen Kommunikationsanbindung, technische Geräte(-Betriebs)-Daten.

Die derart sicher erfassten und gespeicherten Dokumentationsdaten (im Folgenden auch Daten genannt) ermöglichen eine Big Data Analyse. Die Big Data Analyse kann auf einer anderen computer-gestützten Einheit (z.B. zentral) ausgeführt werden, um mögliche Korrelationen herauszufinden, die Therapie entsprechend anzupassen und/oder, um die Therapie vorhersagen zu können. Vorteilhafterweise ist hierzu die Namensnennung nicht mehr notwendig, eine komplett anonyme Behandlung ist möglich.

Mittels des permanenten und fortlaufenden Abgleichs mit Realdaten kann in einer Regelschleife das medizintechnische System zur Durchführung der Behandlung immer besser auf einen optimalen Behandlungserfolg (z.B. Effizienz) konvergieren. Dazu können Methoden der künstlichen Intelligenz, insbesondere Methoden des maschinellen Lernens angewendet werden.

In einer vorteilhaften Weiterbildung des Verfahrens, kann das Verfahren zusätzlich - und insbesondere vor dem Schreiben - ein Validieren zumindest des berechneten zweiten Wertes umfassen. Dazu kann vorzugsweise ein Konsensus-Algorithmus angewendet werden, der dazu dient, die distributed ledger synchronisiert zu halten. Hier kann auf bekannte Algorithmen zurückgegriffen werden. So kann es z.B. konfiguriert sein, dass das Veranlassen der synchronisierten Speicherung nur bei erfolgreicher Validierung ausgeführt wird. Dies hat den Vorteil, dass sichergestellt ist, dass nicht alle intendierten Verwendungen von Verbrauchsgegenständen gespeichert werden, sondern nur die erfolgreich validierten und somit als berechtigt erkannten. Dies kann die anschließenden Auswertungsverfahren (z.B. statistische) beschleunigen, da die Datengrundgesamtheit geringer ist. Alternativ kann nicht nur der zweite Wert, der dem Gebrauchsgegenstand zugeordnet ist validiert werden, sondern alternativ oder kumulativ auch der erste Wert. Damit wird es möglich, gezielte Verbrauchssituationen am Gerät anzusteuern und auszuwerten.

Gemäß einem weiteren bevorzugten Ausführungsbeispiel kann das Verfahren zur Absicherung des Gerätes vor einem nicht autorisierten Betrieb verwendet wird, indem ein Autorisieren des Gerätebetriebs für den ersten und zweiten Verknüpfungspartner nur dann erfolgt, wenn zumindest der berechnete zweite Wert erfolgreich validiert werden konnten. Wenn z.B. automatisch erfasst wird, dass ein Gebrauchsgegenstand bereits für einen anderen Patienten verwendet worden ist, dann schlägt die Validierung fehl und in diesem Fall kann vorkonfiguriert sein, dass das Gerät für genau diese Verwendung mit den beiden Verknüpfungspartnern gesperrt wird und somit vor einem unberechtigten Betrieb abgesichert ist. Weiterhin ist auch die Ausgabe eines entsprechenden Hinweises (z.B. auf einer Benutzeroberfläche mit dem Hinweis auf die wiederholte Verwendung des Disposables) möglich. Umgekehrt kann das Gerät freigeschaltet werden, wenn der intendierte Betrieb mit den beiden Verknüpfungspartnern als berechtigt überprüft (validiert) werden konnte. Zusätzlich zur Validierung können weitere technische Überprüfungsmaßnahmen angewendet werden, die als Autorisierung zusammengefasst werden, wie z.B. die Überprüfung, ob die erfasste Identität des Patienten und/oder des Gerätes für den intendierten Betrieb berechtigt ist. Diese Autorisierungsprüfungen können im Vorfeld konfiguriert werden und limitieren den Gerätebetrieb bzw. die Verwendung des jeweiligen Gebrauchsgegenstandes weiter. Damit können z.B. Medikationen und Präskriptionen für den Patienten (z.B. Behandlung ist nur zu bestimmten Zeiten zulässig) technisch überprüft und automatisch umgesetzt werden. Durch diese technische Maßnahme kann auch überwacht werden, dass ein Gerät z.B. nur mit einem bestimmten Typ von Gebrauchsgegenstand betrieben werden darf (z.B. nur die Disposables dürfen zulässig verwendet werden, die eine Herstellung in einem bestimmten Land aufweisen und entsprechend gelabelt sind). In einer anderen bevorzugten Ausführungsform der Erfindung kann das Gerät für den intendierten Betrieb bei nicht erfolgreicher Validierung und/oder bei fehlender Autorisierung für den jeweiligen Gebrauchsgegenstand gesperrt werden.

In einer anderen bevorzugten Ausführungsform der Erfindung kann zum Zwecke des Validierens auf zumindest einen Speicher einer verteilten Speicherstruktur zugegriffen werden, um für zumindest den berechneten zweiten Wert, der dem Gebrauchsgegenstand zugeordnet ist, zu prüfen, ob dieser bereits gespeichert ist und bei dem nur verneinendenfalls und/oder bei Unterschreiten einer vordefinierbaren Anzahl von zulässigen Wiederholungswerten ein Validieren erfolgt. Dies ist ein wichtiger Vorteil, da lokal am Gerät eine Aussage darüber möglich ist, ob die Verwendung des Gebrauchsgegenstandes möglicherweise deshalb nicht mehr zulässig ist, weil genau dieser Gebrauchsgegenstand an irgendeinem anderen Gerät an irgendeinem Ort auf der Welt (z.B. Dialysezentrum auf einem anderen Kontinent) bereits derart verwendet worden ist, dass die geplante, erneute Verwendung nicht mehr ausgeführt werden darf. Diese Information kann sogar nur mit einem lokalen Speicherzugriff bereitgestellt werden und sogar selbst dann, wenn die Netzwerkverbindung zu entfernten Geräten nicht verfügbar ist. Dies ist möglich, weil die Speicher der Geräte eine verteilte Datenstruktur bilden, die synchronisiert ist.

In einer weiteren bevorzugten Ausführungsform der Erfindung kann das Validieren und/oder ein Autorisieren des Verknüpfungsereignisses zeitlich getaktet und/oder ereignisbasiert ausgeführt wird. Damit kann auf aktuelle Betriebsbedingungen reagiert werden, so dass z.B. zu besonderen Stoßzeiten weniger Limitierungen für den Gerätebetrieb mit dem Disposable auferlegt werden.

In einer weiteren bevorzugten Ausführungsform der Erfindung kann das synchronisierte Speichern unter Verwendung einer verteilten Speicherstruktur erfolgen, insbesondere einer Distributed Ledger (im Folgenden auch kurz: DL) Struktur; die Begrifflichkeit wird weiter unten bei den Definitionen noch genauer erläutert. Dies hat den Vorteil, dass die Dokumentation und/oder eine Absicherung des Gerätes auch dann möglich ist, wenn die Netzwerkverbindung zu anderen Geräten unterbrochen oder gestört ist.

In einer weiteren bevorzugten Ausführungsform der Erfindung hat der Datenblock ein festes, vordefiniertes Format. In einer bevorzugten Ausführungsform der Erfindung ist dieses Format zweiteilig und umfasst einen ersten Bereich (Header) und einen zweiten Bereich (Footer). In einer alternativen Ausführungsform der Erfindung kann der Bereich auch dreiteilig sein. In dieser alternativen Ausführungsform (dreiteiliger Datenblock) kann der Datenblock einen dritten Bereich (der im Folgenden auch als "Body" bezeichnet wird) umfassen. In einer Weiterbildung der alternativen Ausführungsform kann es vorgesehen sein, dass in diesem dritten Bereich ein Verweis auf vorhergehende Datenblöcke gespeichert ist. Der Verweis kann verschlüsselt sein (z.B. verhasht).

In einer weiteren bevorzugten Ausführungsform der Erfindung umfasst das Verfahren ein Berechnen eines dritten Identifikators als Wiederholungswert. Der dritte Identifikator repräsentiert eine Anzahl an Wiederholungen für ein Verknüpfungsereignis zwischen dem ersten und zweiten Verknüpfungspartner eindeutig, so dass Wiederholungen von Verknüpfungsereignissen differenzierbar dokumentiert werden. Der dritte Identifikator kann vorzugsweise in den ersten Bereich des Datenblocks geschrieben werden. Dies hat den technischen Vorteil, dass die Daten auf elektronische Weise einfacher erfasst und/oder ausgewertet werden können. Zudem wird das Auswerten von Wiederverwendungen und/oder Stand- bzw. Betriebszeiten vereinfacht.

In einer weiteren bevorzugten Ausführungsform der Erfindung wenden alle Geräte, die an dem Dokumentationsverfahren bzw. an dem System teilnehmen, dieselben Rechenvorschriften zur Berechnung des ersten Wertes und des zweiten Wertes an.

Vorstehend wurde die Lösung der Aufgabe anhand des Verfahrens beschrieben. Dabei erwähnte Merkmale, Vorteile oder alternative Ausführungsformen sind auch auf die anderen beanspruchten Gegenstände zu übertragen und umgekehrt. Mit anderen Worten können auch die gegenständlichen Ansprüche (die beispielsweise auf ein System oder auf ein Gerät oder ein Dokumentationsmodul gerichtet sind) die Merkmale umfassen, die im Zusammenhang mit dem Verfahren beschrieben und/oder beansprucht sind und umgekehrt. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Das vorstehend in Bezug auf das Verfahren Gesagte mit den Vorteilen und den alternativen Ausführungsform der Erfindung und genannten Merkmalen, gilt ebenso entsprechend auch für die anderen beanspruchten Gegenstände, wie z.B. das Dokumentationsmodul. Auch dieses kann als Softwaremodul, z.B. als Teil einer virtuellen Maschine, ausgebildet sein.

In einem weiteren Aspekt bezieht sich die Erfindung auf ein Computerprogramm mit Programmcode, der für das Ausführen eines Verfahrens wie vorstehend beschrieben geeignet ist, wenn das Computerprogramm auf einem Computer oder einer Computer-basierten Verarbeitungseinheit eines Gerätes oder einem Dokumentationsmodul ausgeführt wird. Das Computerprogramm kann in einem computerlesbaren Medium gespeichert sein. Das Computerprogramm kann auch über eine Netzwerkverbindung zum Download bereitgestellt sein.

Eine weitere Aufgabenlösung sieht ein Computerprogrammprodukt vor, mit Computerprogrammcode zur Durchführung aller Verfahrensschritte des oben näher beschriebenen Verfahrens, wenn das Computerprogramm auf einem Computer ausgeführt wird. Dabei ist es auch möglich, dass das Computerprogramm auf einem von einem Computer lesbaren Medium gespeichert ist. Das Computerprogrammprodukt kann z.B. als gespeicherte, ausführbare Datei, ggf. mit weiteren Bestandteilen (wie Libraries, Treibern etc.) oder als Computer mit dem bereits installierten Computerprogramm ausgebildet sein.

In einem weiteren Aspekt bezieht sich die Erfindung auf ein Dokumentationsmodul für ein Gerät für das medizinische Umfeld, umfassend:

- eine Einleseschnittstelle zum Einlesen eines ersten Identifikators und eines zweiten Identifikators;
- eine elektronische Verarbeitungseinheit, die ausgebildet ist, eine Applikation zur Durchführung des Verfahrens auszuführen;
- eine Schnittstelle zu Speicherstrukturen (umfassend rein lokale Speicher und Speicher, die Bestandteil der distributed ledger sind) zur Speicherung des beschriebenen Datenblocks;
- einer Schnittstelle zu dem Netzwerk, über das die Geräte in Datenaustausch stehen.

In einer bevorzugten Ausführungsform der Erfindung kann das Dokumentationsmodul mit einer Schnittstelle zu weiteren Dokumentationsmodulen von anderen Geräten und/oder Teilnehmern des Dokumentationsverfahrens ausgebildet sein. Dies ermöglicht es, dass die verteilte Datenstruktur synchron gehalten wird.

In einem weiteren Aspekt bezieht sich die Erfindung auf ein Gerät mit einem solchen Dokumentationsmodul.

In einer bevorzugten Ausführungsform der Erfindung umfasst das Gerät einen Speicher, der Bestandteil einer verteilten Datenstruktur ist, die auf mehreren Speichern von unterschiedlichen Geräten ausgebildet ist. Der Speicher dient insbesondere zur Speicherung von Datensätzen in einem festen Format, nämlich zur Speicherung von mehreren Ketten von Blöcken.

In einem weiteren Aspekt bezieht sich die Erfindung auf ein System zur Dokumentation eines Gerätebetriebs eines Gerätes im medizinischen Umfeld, wobei der Gerätebetrieb mit einem Verknüpfungsereignis zwischen zwei Verknüpfungspartnern einhergeht, wobei einer der Verknüpfungspartner ein Gebrauchsgegenstand ist, der bei dem Gerätebetrieb verwendet werden soll bzw. wird. Das Gerät kann in einem verteilten Geräteverbund betrieben werden. Das Gerät umfasst ein Dokumentationsmodul, wie vorstehend beschrieben.

In einer bevorzugten Ausführungsform der Erfindung bilden die Speicher aller oder ausgewählter Geräte eine verteilte Speicherstruktur.

In einer ersten Ausführungsform der Erfindung bezieht sich das System auf ein Dialysedokumentationssystem, bei dem ein erster Verknüpfungspartner ein Patient ist, dem ein ihn eineindeutig identifizierender erster Identifikator zugeordnet ist und bei dem ein zweiter Verknüpfungspartner ein medizinischer Gebrauchsgegenstand ist, der mit einer physikalischen Kennzeichnung (Label, z.B. RFIID-Tag, Barcode etc.) gekennzeichnet ist. Der Kennzeichnung ist auf eineindeutige Weise ein zweiter (digitaler) Identifikator zugeordnet. Das Verknüpfungsereignis repräsentiert auf digitale Weise die Verwendung des jeweiligen Gebrauchsgegenstandes für den jeweiligen Patienten bei einer Behandlung an einem bestimmten medizinischen Gerät.

In einer zweiten Ausführungsform der Erfindung bezieht sich das System auf ein Umkehrosmoseanlagensystem, bei dem ein erster Verknüpfungspartner eine Umkehrosmose-Anlage ist, wobei die Umkehrosmose-Anlage mit einer sie eineindeutig identifizierenden Kennzeichnung ausgebildet ist, wobei der Kennzeichnung auf eineindeutige Weise ein erster Identifikator zugeordnet ist und bei dem ein zweiter Verknüpfungspartner eine Membran ist, die mit einer sie eindeutig identifizierenden Kennzeichnung (Label, wie oben) gekennzeichnet ist, der auf eineindeutige Weise ein zweiter Identifikator zugeordnet ist und bei dem das Verknüpfungsereignis die Verwendung der Membran in der Umkehrosmose-Anlage repräsentiert.

Vorzugsweise ist es sichergestellt, dass allen teilnehmenden Geräten die Rechenvorschriften bekannt sind. Deshalb ist in einer bevorzugten Ausführungsform der Erfindung eine Vorbereitungsphase vorgesehen, die dazu dient, die verwendeten Rechenvorschriften, umfassend die erste, zweite und ggf. dritte auszutauschen, um die Geräte in die Lage zu versetzen, Daten in die verteilte Datenstruktur schreiben und auslesen zu können.
Das Dokumentationsverfahren ist effektiv, kann auf verteilten Smart Devices (z.B. IoT Devices, Iot: Internet of Things) ohne Notwendigkeit einer zentralen Instanz ausgeführt werden und weist zudem eine hohe Manipulationssicherheit auf.

Das Validieren des berechneten Dokumentationswertes kann auf einer konfigurierbaren Gruppe von Geräten ausgeführt werden. Die Gruppe wird bevorzugt aus einer Mehrzahl der Geräte gebildet. Es kann konfiguriert sein, dass grundsätzlich alle Geräte für die Validierung verwendet werden oder nur ausgewählte (z.B. mit ausreichender Rechenleistung).

Die Verknüpfungspartner sind mit physikalischen Kennzeichnungen ausgebildet bzw. mit Labels gekennzeichnet, über die sie eineindeutig identifiziert werden können. Die Kennzeichnungen können z.B. als Code (z.B. als Barcode oder QR-Code) ausgebildet sein. Diese Kennzeichnung ist auf eineindeutige Weise einem Identifikator zugeordnet oder kann in diesen mittels einer Abbildungsvorschrift transformiert werden. Der Identifikator kann einem Gerät in dem Netzwerk auf geeignete Weise zur Verfügung gestellt und übermittelt werden.

In einer Ausführungsform der Erfindung kann der Verknüpfungspartner (z.B. Patient) zumindest zeitweise einer elektronischen Vorrichtung (Handy/Smartphone des Patienten) zugeordnet sein, in der die jeweilige digitale Repräsentation oder Instanz der den Verknüpfungspartner identifizierenden Kennzeichnung hinterlegt ist (z.B. Patienten-ID in einer App). Diese digitale Repräsentation kann dann als Patienten-Identifikator dem Gerät zur weiteren Verarbeitung übermittelt werden.

In einer weiteren Ausführungsform der Erfindung kann zum Beispiel ein Gebrauchsgegenstand mit einer physikalischen Kennzeichnung (z.B. Barcode oder RFID-Chip) versehen sein. Diese Kennzeichnung kann über ein entsprechendes Lesemittel (Scanner, z.B. Barcodescanner oder RFID-Reader) eingelesen und in eine digitale Instanz transformiert werden, die fortan als digitaler Identifikator für den Gebrauchsgegenstand fungiert. Das Lesemittel kann in das Gerät (z.B. Dialysegerät) integriert oder diesem zugeordnet sein. Es ist auch möglich, einen den jeweiligen Verknüpfungspartner identifizierenden Identifikator direkt und manuell (z.B. über eine Benutzerschnittstelle) an dem Gerät einzugeben.

Die Verknüpfungspartner senden 'ihren' Identifikator über ein vorzugsweise kabelloses Netzwerk (z.B. Funk, WLAN, optisch etc.) an ein Gerät des Netzwerkes. Der Verknüpfungspartner (z.B. das Disposable) kann seine physikalische Kennzeichnung z.B. als Barcode an ein Lesemittel bzw. Einlesevorrichtung (z.B. Barcodescanner oder anderweitiger Scanner) senden, die den eingelesenen Code in einen digitalen Identifikator überführt und an das Dialysegerät sendet.

In dieser Ausführungsform der Erfindung können somit zwei unterschiedliche Netzwerke implementiert sein: Ein (erstes) Netzwerk zwischen den bei dem Gerätebetrieb zu verknüpfenden Verknüpfungspartnern (z.B. Disposable mit RFID Chip) und deren zugeordneten Vorrichtungen bzw. Lesemitteln (z.B. RFID Scanner für den RFID-CHip) und ein anderes (zweites) Netzwerk, das zum Datenaustausch zwischen den Geräten dient, auf denen das Dokumentationsverfahren angewendet werden soll (Dialysegerät oder Gateway) und deren Speicher eine verteilte Datenstruktur bilden. Letzteres kann insbesondere auf einem IP-Protokoll basieren.

Im Folgenden werden die Begrifflichkeiten der Anmeldung und vorteilhafte Ausführungsformen der Erfindung spezifiziert.

Ein Gerät kann ein medizinisches Gerät sein (z.B. ein Dialysegerät) oder ein nichtmedizinisches, das im medizinischen Umfeld verwendet wird (z.B. eine Umkehrosmose-Anlage, die im Folgenden aufgrund des englischen Begriffs "reverse osmosis" kurz als RO-Anlage bezeichnet wird) oder ein Gerät, das im Rahmen einer therapeutischen Apherese mit einem Gebrauchsgegenstand betrieben wird. Bei dem medizinischen Gerät kann es sich z.B. um ein Hämodialysegerät, ein Hämodiafiltrationsgerät oder ein Peritonealdialysegerät handeln. Das Dialysegerät wird vorzugsweise in einem Verbund von Geräten betrieben. So kann das Dialysegerät mit anderen parallel in einem Dialysezentrum betrieben werden. Das Dialysegerät kann aber auch als Heimdialysegerät betrieben werden. Dabei werden weltweit unterschiedliche Dialysezentren betrieben. Ein wesentlicher Vorteil der vorgeschlagenen Lösung ist darin zu sehen, dass die Geräte von unterschiedlichen Herstellern stammen und/oder von unterschiedlichen Betreibern betrieben werden können, um an dem Dokumentationsverfahren teilnehmen zu können.

Das Gerät kann auch ein Heim-Hämodialysegerät sein. Gerade dort ist eine Wiederverwendung eines Dialysators aus wirtschaftlichen Gründen geradezu notwendig. Auf der anderen Seite ist der Patient aber weitgehend ohne Aufsicht von medizinischem Fachpersonal. Dort könnte also leichter eine Überschreitung der gesundheitlich zulässigen Wiederholungszahl unbemerkt auftreten (oder aus Kostengründen sogar beabsichtigt), so dass die erfindungsgemäße Absicherung bzw. Überprüfung dahingehend, dass nicht zu viele Wiederholungen auftreten, äußerst hilfreich ist. Zugleich erweist es sich für Krankenkassen und Herstellerunternehmen als wünschenswert, dass sichergestellt werden kann, dass es fälschungssicher dokumentiert wird, wenn eine unzulässige Wiederholungszahl auftritt. So können ggf. Haftungsfragen geklärt und das Einhalten von Behandlungsplänen bezeugt werden.

Ein Gebrauchsgegenstand kann als Einwegoder Mehrwegartikel für ein Dialysegerät ausgebildet sein, wie z.B. einen Dialysator, eine Blutleitung oder eine Dialysatleitung, die als Schlauchleitungen ausgebildet sein können. Der Gebrauchsgegenstand kann ebenso eine Membran oder einen anderen Gebrauchsgegenstand für eine Umkehrosmoseanlage betreffen. In einem weiteren Anwendungsbeispiel kann der Gebrauchsgegenstand auch ein Dialysefilter wie ein Dialysator für Hämodialyse und/oder Hämodiafiltration sein. In einem weiteren Anwendungsbeispiel kann der Gebrauchsgegenstand auch ein Adsorber für eine therapeutische Apherese sein. Der Gebrauchsgegenstand kann in vorteilhaften Weiterbildungen der Erfindung auch ein sonstiger Gegenstand oder ein weiteres Gerät sein, das an oder mit dem Gerät verwendet und/oder betrieben wird. In einer bevorzugten Ausführungsform der Erfindung ist der Gebrauchsgegenstand ein Verbrauchsgegenstand, insbesondere ein medizinisches Einmalprodukt (Disposable).

Die Autorisierung basiert auf einer automatischen Überprüfung der Zulässigkeit einer Verwendung eines Gebrauchsgegenstandes für das medizintechnische Gerät und/oder für einen Patienten. Dabei können je nach Anwendung unterschiedliche Zulässigkeitsvoraussetzungen konfiguriert werden, wie z.B. nur einmalige Verwendung oder mehrfache Verwendung des Gebrauchsgegenstandes mit einer konfigurierbaren Begrenzung der Anzahl von zulässigen Verwendungen oder anderen Zulässigkeitskriterien (z.B. maximale Standzeit, maximale Betriebsdauer etc.) für den jeweiligen Verknüpfungspartner (z.B. Dialysemaschine). Die Autorisierung kann eine Freischaltung des Gerätes und/oder eine Sperrung für den geplanten Betrieb des Gerätes mit dem Gebrauchsgegenstand umfassen.

Die Autorisierung kann noch weitere Zulässigkeitprüfungen umfassen (z.B. siehe oben: Die Überprüfung von Zulässigkeitsvoraussetzungen).

Ein erster Verknüpfungspartner kann ein Patient sein, der an einem Dialysegerät behandelt wird und ein zweiter Verknüpfungspartner kann ein Gebrauchsgegenstand (Dialysator, Schlauchset für den extrakorporalen Blutkreislauf etc.) zur Verwendung mit dem Dialysegerät sein. Alternativ zur Ausbildung als Dialysegerät kann der erste Verknüpfungspartner auch eine Umkehrosmoseanlage und der zweite Verknüpfungspartner eine Membran oder ein anderer Gebrauchsgegenstand für die Umkehrosmoseanlage sein. Ein Verknüpfungspartner kann eine ihn eineindeutig identifizierende Kennzeichnung (z.B. in Form eines QR-Codes) aufweisen. Diese Kennzeichnung ist auf eineindeutige Weise einem digitalen Identifikator zugeordnet. So hat z.B. jeder Patient einen ihn identifizierenden Patienten-Identifikator und jeder Gebrauchsgegenstand einen ihn identifizierenden Disposable-Identifikator.

Die Geräte können Kommunikationspartner in einem Netzwerk sein, die über ein vorzugsweise drahtloses Netzwerk (z.B. WLAN, nach einem Protokoll gemäß dem Standard der IEEE-802.11-Familie) in Datenaustausch stehen und insbesondere Daten in eine verteilte Speicherstruktur schreiben und lesen können.

Der erste und zweite Identifikator dienen jeweils zur eindeutigen Identifikation des ersten bzw. zweiten Verknüpfungspartners. Bei dem Identifikator handelt es sich vorzugsweise um einen digitalen bzw. elektronisch verarbeitbaren Identifizierungscode, der einer direkt auf dem Verknüpfungspartner (Gebrauchsgegenstand ist gekennzeichnet) aufgebrachten oder diesem zugeordneten (Patient hat eine Patientenkarte, die ihn identifiziert) Kennzeichnungen bijektiv zugeordnet ist. Der Identifikator kann z.B. über einen Zufallsgenerator erzeugt und mit einem Algorithmus derart bearbeitet sein, dass sichergestellt ist, dass eine eineindeutige Zuordnung zwischen Identifikator und Verknüpfungspartner gewährleistet ist, so dass genau ein Identifikator genau einem Verknüpfungspartner zugeordnet ist.

Die Kennzeichnung ist ein physikalisches Label, Tag oder Token und kann direkt auf dem Gebrauchsgegenstand aufgebracht sein, z.B. als aufgeklebtes elektronisches Label. Eine Kennzeichnung kann beispielsweise Zahlen, Buchstaben, Sonderzeichen sowie Kombinationen aus diesen umfassen. Die Kennzeichnung kann auch einem Verknüpfungspartner eineindeutig zugeordnet sein. Die Kennzeichnung wird über entsprechende Schnittstellen bzw. Einlesemittel erfasst bzw. eingelesen (Datenschnittstelle oder optischer Sensor, Scanner). Die Kennzeichnung kann eine Kennung sein, die zur eindeutigen Identifizierung des Verknüpfungspartners dient (Barcode, QR-Code) und ist elektronisch erfassbar und auswertbar. Wenn die Kennzeichnung eingelesen ist, dann kann sie mittels einer eineindeutigen Abbildungsvorschrift einem Identifikator zugeordnet sein.

Ein Verknüpfungsereignis repräsentiert die Verknüpfung von zumindest zwei Verknüpfungspartnern. Mit "Verknüpfung" ist hier ein gemeinsamer Betrieb oder die Verwendung eines Gebrauchsgegenstands durch einen Verbraucher bei bzw. während einem/eines Betrieb(s) gemeint. Das Gerät kann bei dem Verknüpfungsereignis mitwirken. Das Verknüpfungsereignis kann während einer Aufrüstung, Vorbereitung, Inbetriebnahme und/oder während des Betriebs des Gerätes erfolgen. Mit anderen Worten repräsentiert die Verknüpfung z.B. die Verwendung eines bestimmten Dialysators oder Schlauchsets als Gebrauchsgegenstand in dem Dialysegerät für den bestimmten Patienten oder die Verwendung der bestimmten Membran in der Umkehrosmoseanlage. Zunächst wird ein Verknüpfungsereignis intendiert oder angefragt von zumindest einem der Verknüpfungspartner. Dies kann z.B. ausgelöst werden, indem der Patient seine ID-Karte in eine Aufnahmevorrichtung an dem Gerät einführt und/oder entsprechende Benutzereingaben auf einer Benutzeroberfläche eingibt und/oder indem ein Identifikator des dabei zu verwendenden Gebrauchsgegenstandes eingelesen wird. Um die Sicherheit zu erhöhen, wird das Verknüpfungsereignis zumindest fälschungssicher abrufbar dokumentiert und gespeichert. Des Weiteren kann es vorgesehen sein, dass die Verknüpfung nicht gleich umgesetzt wird, sondern dass zunächst eine Überprüfung erfolgt, ob die geplante Verwendung auch autorisiert ist. Dazu wird auf die verteilte Speicherstruktur mit einem definierten Zugriffsalgorithmus zugegriffen. Ein Verknüpfungsereignis kann auf Basis von konfigurierbaren Kriterien erfasst werden. Eine Erfassung eines Verknüpfungsereignisses kann zeitbasiert (z.B. zeitlich getaktet erfolgen oder nach einer bestimmten Anzahl von Verwendungen) oder ereignisbasiert (z.B. nach dem Einlegen oder dem Ausführen einer bestimmten Handlung/Aktion) erfolgen.

Bei der ersten, zweiten, dritten und/oder weiteren Rechenvorschrift und/oder der Verknüpfungsfunktion kann es sich um eine auf einer digitalen Recheneinheit auszuführende Vorschrift handeln. In einer bevorzugten Ausführungsform der Erfindung sind diese Rechenvorschriften identisch. Sie können aber auch unterschiedlich ausgebildet sein. Die erste Rechenvorschrift wird auf den ersten Identifikator angewendet, um einen ersten Wert zu berechnen und die zweite Rechenvorschrift wird auf den zweiten Identifikator angewendet, um den zweiten Wert zu berechnen. Der erste und der zweite Wert repräsentieren auf sichere Weise ein Verknüpfungsereignis. Die Rechenvorschrift kann insbesondere kryptologisch sein. Die Rechenvorschrift kann z.B. eine kryptologische Hashfunktion oder eine einfache Hashfunktion sein, wie z.B. die Anwendung eines SHA, MD5 oder anderen Algorithmus.

Der Datenblock ist eine Datenstruktur mit festem Format. Sie besteht aus einem ersten und einem zweiten Datenbereich, die jeweils zur Speicherung von dem ersten Wert (Anwendung der ersten Rechenvorschrift auf den ersten Identifikator) und dem zweiten Wert (Anwendung der zweiten Rechenvorschrift auf den zweiten Identifikator) bestimmt sind. In alternativen Ausführungsformen der Erfindung kann der Datenblock ein dreielementiges Format aufweisen und zusätzlich einen Body umfassen, in dem z.B. Verweise auf vorhergehende Blöcke gespeichert sein können. Auf den ersten und/oder den zweiten Wert kann eine dritte Rechenvorschrift angewendet werden. Alle Teilnehmer des Dokumentationsverfahrens einigen sich im Vorfeld (in einer Konfigurationsphase) auf ein einheitliches Format für die Datenstruktur (mit 2 oder 3 Bereichen).

In bevorzugten Ausführungsformen der Erfindung kann der erste und/oder der zweite Wert und/oder gegebenenfalls der Body (dritter Bereich des Datenblockes) darüber hinaus noch weitere Daten, insbesondere Messdaten, die während der Geräteverwendung mit den Verknüpfungspartnern (z.B. mit dem jeweiligen Disposable während einer Dialysebehandlung) anfallen, umfassen. Weitere Daten, die mit dem erfindungsgemäßen Verfahren sicher dokumentiert werden, können sein (dabei können alle der im Folgenden genannten Datenkategorien oder nur ausgewählte zur sicheren Dokumentation bestimmt werden) :

- Geräte ID, Komponenten IDs, Standortdaten, Behandlungsdaten wie mittlerer Blutdruck, Clearance, mittlere Leitfähigkeit des Dialysats (Na Konzentration), etc. und/oder
- Information zur Kommunikationsanbindung (Latenz, IP Adresse, Mac Adresse, physikalische Location) - das Tracking dieser Daten hat den technischen Vorteil, dass die Kommunikationsverbindung in ihrer Güte vorhersagbar und/oder das System damit insgesamt zuverlässiger ausgebildet werden kann; und/oder
- Ausführungsdauer des erfindungsgemäßen Verfahrens auf diesem Gerät; dabei kann die Ausführungsdauer für verschiedene Hardware unterschiedlich sein - und der Vorteil ist in der verbesserten Vorhersagbarkeit begründet;
- Die vorstehend genannte Liste von weiteren Daten ist erweiterbar. Im Grunde sind alle Behandlungs- sowie Umgebungsdaten denkbar, wie z.B. Blutdruck sowie Medikamentengabe wie Heparin, Epo etc. Zudem können Alarme Teil der weiteren Daten sein.

Die Speicherung der weiteren Daten (wie vorstehend genannt) dient zum anonymisierten Datentracking unter Einhaltung der besonderen Bestimmungen für Medizindaten, die sicherstellen, dass keine Patienten-bezogenen Daten die informationstechnologische Domäne des Patienten ohne zusätzliche Sicherungsmaßnahmen verlassen und/oder aus anderen Daten abgeleitet werden können. Das anonymisierte Datentracking soll generell dazu verwendet werden, durch computer-implementierte Algorithmen zur Datenanalyse einer Vielzahl von Behandlungen Erkenntnisse zu gewinnen und so den Behandlungserfolg zu verbessern und/oder den Behandlungserfolg vorhersagen zu können.

Für die Effektivität einer Dialysebehandlung ist von entscheidender Bedeutung die sogenannte Dialysedosis K·T/V, die als der Quotient aus dem Produkt von Clearance K für Harnstoff und effektiver Behandlungszeit T der Dialysebehandlung und dem Verteilungsvolumen V des Patienten für Harnstoff definiert ist..

Derartige Verbesserungen des Behandlungserfolges können umfassen:

- Veränderte Maschineneinstellung; dies führt zu einer Therapieveränderung oder auch zur Ausführung alternativer Therapien, bspw. statt Hämodialyse -> Hämodiafiltration; die geänderte Maschineneinstellung kann auch eine Veränderung der Substitutionsrate bzw. des Zugabeortes der Substitutionslösung in den extrakorporalen Blutkreislauf (beispielsweise von flussabwärts des Dialysators zur flussaufwärts oder zu sowohl flussabwärts als auch flussaufwärts mit variablen Raten) zur Folge haben;
- alternative Dialysatkompositionen: diese können im Gerät online angemischt werden, ein Feedback würde die Komposition ändern; alternativ kann auch auf unterschiedliche fertig angemischte Dialysatkompositionen zurückgegriffen werden; die Zusammensetzung des Dialysats hat direkten Einfluss auf die Clearance-Leistung des Dialysators und damit auf den Behandlungserfolg;
- geänderte Behandlungszeiten, beispielsweise um den Patienten zu schonen, wenn eine Erkenntnis aus der "BigData" Analyse ist, dass unter bestimmten Voraussetzungen die Behandlungsdauer reduziert werden kann, ohne den Behandlungserfolg wesentlich einzuschränken.

Um derartige Therapieeinstellungen beim konkreten Patienten durchführen zu können, sind auch anonymisierte Daten des Patienten als getrackte Daten hilfreich. In einer bevorzugten Ausführungsform der Erfindung umfassen die weiteren Daten somit: Geschlecht, Alter, Nass/Trockengewicht (also das Körpergewicht vor und nach der Dialysebehandlung), Rasse etc. Im Prinzip können alle Daten getrackt (auf sicheren Weise gespeichert) werden, die zur Kategorisierung von unterschiedlichen Behandlungsprozessen dienen. Werden in einer Kategorie Verbesserungsoptionen durch die Ausführung der algorithmische Analyse festgestellt, so ist es mit dem Verfahren möglich, diese Verbesserungsoptionen unmittelbar und ohne weitere Bearbeitungsschritte auch anderen Patienten zu ermöglichen, die der gleichen Kategorie angehören.

Dazu werden vorzugsweise alle oder ausgewählte Behandlungsdaten getrackt, die im Folgenden beschrieben werden:

- Therapieform (Hämodialyse, Hämofiltration, Hämodiafiltration),
- Behandlungsdauer,
- Dialysatkomposition (Leitfähigkeit, insbesondere gemessen vor und nach dem Dialysator gemessen),
- Ultrafiltrationsrate und -volumen (auch Verlauf über bspw. bestimmte zeitabhängige Profile),
- Blutrate (auch Verlauf über bspw. bestimmte zeitabhängige Profile),
- Substitutionsrate und Zugabeort (downstream upstream beides/),
- gemessener Blutdruck (vor, während, nach der Therapie),
- Events (Alarme wegen Grenzwertverletzung, Abbruch der Behandlung wegen eines medizinischen Vorfalls, bspw. Kreislaufentgleisung, etc.),
- Bluttemperatur, Körpertemperatur;
- Weitere während der Behandlung des Patienten anfallenden Daten;
- Maschinen- ID (und damit Typ der Maschine),
- Laufzeit der Dialysemaschine (die Maschinen können über einen Laufzeitzähler verfügen),
- Ausrüstung der Maschine (bspw. optionale Ausstattung),
- Standort (daraus ist indirekt ermittelbar ein Datensatz zu Patienten-spezifischen Metadaten, bspw. Essgewohnheiten, die sich aus dem Standort typischerweise ergeben);
- Umgebungsvariablen, wie Uhrzeit der Behandlung, Datum (Klima, Wetter), Umgebungstemperatur.

Bei dem Netzwerk handelt es sich um ein Netzwerk zum Austausch von digitalen Daten zur Ausführung des Dokumentationsverfahrens. Das Netzwerk kann vorzugsweise als Peer-to-Peer-Netzwerk ausgebildet sein und bietet den Kommunikationsteilnehmern die Möglichkeit, Dienste und Ressourcen von den anderen Kommunikationsteilnehmern auf gleichberechtigte Weise in Anspruch zu nehmen. Das Netzwerk ist offen für alle Netzwerkteilnehmer. Dies stellt kein Sicherheitsrisiko dar, da die in der verteilten Speicherstruktur abgelegten Daten gesichert abgelegt und vorzugsweise kryptologisch verschlüsselt sind und somit selbst bei einem Abhören nicht im Klartext vorliegen.

Unter einem "Programm" bzw. "Programminstruktionen" wird hier jede Art von Computerprogramm verstanden, das maschinenlesbare Instruktionen zur Steuerung einer Funktionalität des Computers umfasst.

Das Computerprogramm kann auf einem Datenträger als ausführbare Programmdatei, häufig im sogenannten Maschinencode gespeichert sein, die zur Ausführung in den Arbeitsspeicher des Rechners geladen wird. Das Programm wird als Abfolge von Maschinen-, d. h. Prozessorbefehlen von dem oder den Prozessoren des Computers verarbeitet und damit ausgeführt. Das Programm kann als ausführbarer Code, als Quellcode oder als interpretierter Code vorliegen.

Unter einer "Schnittstelle" wird eine Schnittstelle verstanden, über die Daten empfangen und gesendet werden können (Kommunikationsschnittstelle). Die Kommunikationsschnittstelle kann kontaktbehaftet oder kontaktlos konfiguriert sein. Bei der Kommunikationsschnittstelle kann es sich um eine interne Schnittstelle oder um eine externe Schnittstelle handeln, die beispielsweise mittels eines Kabels oder kabellos mit einem zugeordneten Gerät verbunden ist. Die Kommunikation kann über ein Netzwerk erfolgen. Unter einem "Netzwerk" wird hier jedes Übertragungsmedium mit einer Anbindung zur Kommunikation verstanden, insbesondere eine lokale Verbindung oder ein lokales Netzwerk, insbesondere ein Local Area Network (LAN), ein privates Netzwerk, insbesondere ein Intranet, und ein virtuelles privates Netzwerk (Virtual Private Network - VPN). Beispielsweise kann ein Computersystem eine Standardfunkschnittstelle zur Anbindung an ein WLAN aufweisen. Ferner kann es sich um ein öffentliches Netzwerk, wie beispielsweise das Internet handeln. Je nach Ausführungsform kann die Kommunikation auch über ein Mobilfunknetz erfolgen.

Unter einem "Speicher" werden hier sowohl flüchtige als auch nichtflüchtige elektronische Speicher bzw. digitale Speichermedien verstanden. Unter einem "nichtflüchtigen Speicher" wird ein elektronischer Speicher zur dauerhaften Speicherung von Daten verstanden. Ein nichtflüchtiger Speicher kann als nichtänderbarer Speicher konfiguriert sein, der auch als Read-Only Memory (ROM) bezeichnet wird, oder als änderbarer Speicher, der auch als Non-Volatile Memory (NVM) bezeichnet wird. Insbesondere kann es sich hierbei um ein EEPROM, beispielsweise ein Flash-EEPROM, kurz als Flash bezeichnet, handeln. Ein nichtflüchtiger Speicher zeichnet sich dadurch aus, dass die darauf gespeicherten Daten auch nach Abschalten der Energieversorgung erhalten bleiben. Unter einem "flüchtigen elektronischen Speicher" wird hier ein Speicher zur vorübergehenden Speicherung von Daten verstanden, welcher dadurch gekennzeichnet ist, dass alle Daten nach dem Abschalten der Energieversorgung verloren gehen. Insbesondere kann es sich hierbei um einen flüchtigen Direktzugriffsspeicher, der auch als Random-Access Memory (RAM) bezeichnet wird, oder einen flüchtigen Arbeitsspeicher des Prozessors handeln. Vorzugsweise sind zwei unterschiedliche Speicherstrukturen vorgesehen: eine lokale Speicherstruktur und eine Speicherstruktur, die Bestandteil einer distributed ledger sein kann. Die lokale Speicherstruktur kann als geräteinterner Zwischenspeicher oder Pufferspeicher vor die dezentrale distributed ledger Struktur geschaltet sein, so dass der Block zunächst in den lokalen Speicher geschrieben wird und erst nachfolgend, insbesondere nach erfolgreicher Validierung (die ein zulässiges Pairing von den beiden Verknüpfungspartners signalisiert), in die verteilte Speicherstruktur gespeichert wird. In diesem Fall bezieht sich "Schreiben" somit auf eine lokale geräteinterne Speicherung und "synchronisierte Speicherung" auf eine Speicherung in der dezentralen geräte-übergreifenden Speicherstruktur, auf die auch andere Geräte und/oder Teilnehmer des Dokumentationsverfahren (mit einem Schreib- und/oder Lesezugriff) zugreifen können.

Unter einer "Verarbeitungseinheit" wird ein Elektronikmodul verstanden. Es handelt sich typischerweise um eine digitale Verarbeitungseinheit, die als Softwaremodul implementiert sein kann, z.B. als Teil von virtuellen Maschinen. Die Verarbeitungseinheit kann z.B. als Prozessor zur computerbasierten, automatischen Ausführung von Befehlen ausgebildet sein und eine Logikschaltung zur Ausführung von Programminstruktionen umfassen. Die Logikschaltung kann auf einem oder mehreren diskreten Bauelementen implementiert sein, insbesondere auf einem Chip. Insbesondere wird unter einem "Prozessor" ein Mikroprozessor oder ein Mikroprozessorsystem aus mehreren Prozessorkernen und/oder mehreren Mikroprozessoren verstanden.

In einer bevorzugten Ausführungsform der Erfindung kann der erste und/oder zweite Wert zusätzlich weitere Informationen zu dem jeweiligen Verknüpfungsereignis bereitstellen, die z.B. eine Aussage darüber zulassen, ob die Aufrüstung der Dialysemaschine ausreichend und angepasst ist, mit den jeweiligen Teilen des extrakorporalen Kreislaufs in Eingriff gebracht werden zu können. Darüber hinaus kann der Wert noch weitere Metadaten zu dem Verknüpfungsereignis bereitstellen (Zeitpunkt, Dauer, Anzahl von bisherigen Verwendungen, ggf. Wiederholungsrate, Anzahl verbleibender Verwendungen etc.).

Ein Konsensus-Algorithmus wird verteilt auf mehreren Knoten bzw. Geräten ausgeführt und dient zur Herstellung eines Konsenses bzw. einer Übereinstimmung in Bezug auf einen Wert und damit zum Validieren des Wertes. Er wird auf allen oder ausgewählten Geräten oder Teilnehmern des Dokumentationsverfahrens einheitlich ausgeführt, um eine übereinstimmende Beurteilungsgrundlage zu schaffen. Der Konsensus-Algorithmus wird nach nach einem Konsens-Protokoll betrieben und kann als Proof-of-Work- oder (delegated) Proof-of-Stake-Algorithmus ausgebildet sein. Ein Konsensus-Algorithmus bezieht sich grundsätzlich auf einen Mechanismus zum Erreichen einer einheitlichen Vereinbarung (Konsens) über den Zustand (Status) des dezentralen Netzes. Ein Konsens-Algorithmus erleichtert die Überprüfung und Validierung von Informationen, die in die verteilte Datenstruktur (DL, Hauptbuch) geschrieben (gespeichert) werden. Dadurch wird sichergestellt, dass nur authentische Daten in der verteilten Datenstruktur aufgezeichnet werden. Die Regeln zur Anwendung in dem Konsens-Protokoll sind hart codiert, einvernehmlich unter allen Teilnehmern vereinbart und stellen die einzige Quelle von Überprüfungsregeln dar, so dass sichergestellt werden kann, dass die gespeicherten Informationen vertrauenswürdig sind, ohne dass eine zentrale Instanz erforderlich ist. Für weitere Details sei auf das folgende Paper verwiesen "Blockchain consensus protocols in the wild", C Cachin, M Vukolic - arXiv preprint arXiv:1707.01873, 2017 - arxiv.org (v2). Erst wenn ein Dokumentationswert validiert werden konnte, kann er auf synchronisierte Weise in allen Speichern der Verknüpfungspartner, die eine verteilte Speicherstruktur bilden, gespeichert werden. Der Konsensus-Algorithmus umfasst einen Zugriff auf die verteilte Speicherstruktur. Das Validieren mittels des Konsensus-Algorithmus' dient zur lokalen Suche, ob der jeweilige Wert schon einmal und wenn ja, wie oft, abgespeichert worden ist. In einer Erweiterung können dabei noch weitere Schritte ausgeführt werden, wie z.B. die Anwendung von in einer Regelbasis vorgehaltenen Regeln und / oder darauf basierender computer-implementierter Algorithmen. Das Validieren erfolgt ähnlich wie bei der klassischen Blockchain, indem ein Teilnehmer im Netz (Gateway oder Gerät) versucht, einen berechneten Wert (entsprechend bspw. einer Transaktion bei Bitcoin) zu verifizieren, indem versucht wird, dieselbe (identische) Signatur zu erzeugen. Die Suche in der Distributed Ledger kann direkt nach der Signatur erfolgen und ist damit schneller im Vergleich zu einer Suche über Speicheradressen und Blocknamen etc.

Es kann konfiguriert sein, dass ein Gerätebetrieb nur dann aktivierbar ist, wenn er mit den jeweiligen Verknüpfungspartnern als autorisiert evaluiert worden ist. Für die Evaluation ist eine erfolgreiche Validierung (wie vorstehend erläutert auf Basis des berechneten Wertes) eine zwingende Voraussetzung. Für die Autorisierung des Gerätes können unter Umständen noch weitere Prüfungen durchgeführt werden (zeitbasiert, ereignisbasiert etc.). Damit kann der Gerätebetrieb auf flexible und verteilte Weise gegen einen unautorisierten Betrieb abgesichert werden.

Das synchronisierte Speichern bedeutet, dass ein Wert, sofern er erfolgreich validiert werden konnte, in allen Speichern der Geräte des Dokumentationsverfahrens bzw. des Dokumentationssystems identisch gespeichert wird. Die Geräte können z.B. eine Gruppe von Dialysegeräten und/oder Umkehrosmoseanlagen sein. Die Geräte des Validierungsverfahrens müssen nicht notwendigerweise mit den Verknüpfungspartnern übereinstimmen. Damit kann der Kernschritt zur Absicherung des Gerätebetriebs, nämlich die Validierung, nicht direkt auf den Verknüpfungspartnern, sondern auf anderen computer-basierten Knoten bzw. Instanzen ausgeführt werden, z.B. auf Gateways oder den Geräten zugeordneten Knoten. Alle Speicher der Geräte sind immer auf demselben Stand und verfügen über denselben Speicherstatus. Damit entsteht eine verteilte Speicherstruktur.

Bei der verteilten Speicherstruktur handelt es sich vorzugsweise um eine distributed ledger Struktur (DLT-Struktur), die auf allen potentiellen Geräten und Teilenehmern des Dokumentationsverfahrens implementiert sein kann. Die Geräte sind dazu mit Mitteln (darunter Speichermittel und Sofwaremittel) ausgestattet, um an der distributed ledger Struktur teilzunehmen. Ein distributed ledger System (verteiltes Hauptbuchsystem) kann Daten speichern, die auf verschiedene Transaktionen bzw. Berechnungen zwischen bzw. von mehreren Rechengeräten (z.B. mehreren Dialysegeräten) hinweisen. Das verteilte Ledgersystem kann als Blockchain-Ledgersystem ausgeführt werden, das eine Vielzahl von "Blöcken" umfasst, die jeweils eine oder mehrere diskrete Transaktion(en) darstellen, die zwischen den Rechengeräten stattfinden. Jeder Block kann Daten umfassen, die mit einem zuvor erzeugten Block verknüpft sein können, wodurch eine vollständige Kette zwischen der Erzeugung von Daten, die im verteilten Ledger gespeichert sind, und der späteren Verwendung derselben Daten entsteht (z.B. zum Aufbau einer vollständigen Kette von Verwendungen von Verbrauchsgegenständen für einen Patienten in einem Gerät). Die in den verschiedenen Transaktionen/Blöcken gespeicherten Daten können verschlüsselt, mit einem Passwort versehen oder anderweitig vor unbefugtem Zugriff geschützt sein (z.B. Lesezugriff, Schreibzugriff und/oder Löschzugriff). In einem nicht einschränkenden Beispiel können Informationen, die in den verschiedenen Blöcken gespeichert sind, irreversibel gehasht werden, so dass die gehashten Daten verwendet werden können, um die Echtheit von Transaktionen zu überprüfen, wobei sichergestellt ist, dass die gehashten Daten nicht rückentwickelt werden können, um substantielle Informationen allein basierend auf den gehashten Informationen zu ermitteln. Damit können sicherheitskritische medizinische Daten auf sichere Weise verteilt werden. Darüber hinaus können Daten, die zwischen verschiedenen Computern übertragen werden, verschlüsselt werden (z.B. durch Verwendung von öffentlichen/privaten Schlüsselpaaren zum digitalen Signieren und/oder Verifizieren von Daten), so dass innerhalb der Blockkette gespeicherte Blöcke von mehreren Geräten verifiziert werden können, die Zugriff auf die öffentlichen und/oder privaten Schlüssel haben. Nach Überprüfung (Validierung) der in der Blockchain zu speichernden Daten, können die Daten, wie oben beschrieben, gehasht und gespeichert werden. Für weitere Details zu den Begrifflichkeiten verteiltes System / distributed ledger sei auf A. S. Tanenbaum, M. van Steen: Verteilte Systeme, 2007, insbesondere Kapitel 7.5 verwiesen. Auf den einzelnen verteilten Recheninstanzen bzw. Computern laufen Prozesse zur Ausführung des erfindungsgemäßen Dokumentationsverfahrens. Prozesse auf den unterschiedlichen Recheninstanzen in dem verteilten System kommunizieren miteinander durch das Senden von Nachrichten. Dabei müssen die Prozesse Regeln einhalten, z. B. bezüglich der Formate, der Bedeutungen der ausgetauschten Nachrichten und der erforderlichen Aktionen zur Übermittlung. Diese Regeln werden als Protokoll bezeichnet.

Mit dem erfindungsgemäßen Dokumentationsverfahren werden Blöcke (Datenblöcke) in einer Vielzahl von Ketten (Chains) erstellt (im Gegensatz zu Blockchain, wo es typischerweise genau eine Kette gibt). Die Blöcke der Kette bilden eine Datensammlung. Die verteilte Speicherstruktur kann als modifizierte Blockchain Struktur ausgebildet sein. Die Modifikation bezieht sich darauf, dass ein Datenblock gemäß einer grundlegenden Ausführungsform der Erfindung lediglich aus dem berechneten Wert (Hashwert) besteht, der eine einzigartige Signatur darstellt. Ein Datenblock muss keinen Verweis auf die anderen Blöcke der Kette enthalten. Um einen Datenblock auszurechnen muss, im Unterschied zur klassischen Blockchain, nicht auf einen Miningpool zurückgegriffen werden. Die Berechnungen können deutlich schneller und auch auf weniger leistungsfähigen Computereinheiten ausgeführt werden und sind dennoch fälschungssicher hinterlegt. Ein weiterer struktureller Unterschied zur Architektur der Blockchain liegt darin, dass die modifizierte Blockchain Struktur mehrere Ketten umfasst und somit einen Multi-Ketten Datenraum darstellt. Dabei kann sich eine Kette in einer ersten Ausführungsform jeweils auf einen Verknüpfungspartner, z.B. einen Patienten oder eine Umkehrosmoseanlage beziehen. Es können mehrere Ketten (z.B. eine Kette pro Gebrauchsgegenstand oder pro Patient) in einer verteilten Speicherstruktur (z.B. auf einer Gruppe von Dialysegeräten) gespeichert sein. Da ein Patient einerseits seinen Identifikator in der Regel nicht ändert, aber andererseits viele Verbrauchsgüter (die jeweils individuelle Identifikatoren aufweisen) verwendet, sollten viele Ketten pro Patient entstehen - aber nur eine pro Gebrauchsgegenstand.

In einer bevorzugten Ausführungsform der Erfindung wird auf einem Dialysegerät ein Speicher oder ein Speicherbereich bereitgestellt, in dem eine Instanz der verteilten Speicherstruktur abgelegt ist (z.B. als modifizierte Blockchain). Dort können alle auf dem Dialysegerät erfolgten (also validierten) und/oder intendierten (noch nicht validierten) Verknüpfungsereignisse gespeichert werden. Die Verknüpfungsereignisse können sich auf unterschiedliche Verbrauchsgegenstände und/oder auf unterschiedliche Patienten beziehen. Dies hat den technischen Vorteil, dass eine Absicherung mittels einer Validierungsprüfung lokal auf einem Teilnehmer(gerät) ausgeführt werden kann. Insbesondere kann die Validierungsprüfung vorteilhaftweise auch dann ausgeführt werden, wenn zeitweise keine (oder keine ausreichende) Kommunikationsverbindung zu anderen Geräten besteht, da die Speicher der Geräte stets synchronisiert sind.

In einer Ausführungsform der Erfindung umfasst die Berechnung des Wertes das Berechnen eines Wiederholungswertes. Der Wiederholungswert repräsentiert dabei eine Wiederholungsrate oder eine zulässige Anzahl von Wiederholungen für ein Verknüpfungsereignis zwischen dem ersten und zweiten Verknüpfungspartner auf eindeutige Weise. Damit kann auf sichere Weise nachvollzogen und differenzierbar dokumentiert werden, ob und wenn ja, wie viele Wiederholungen von Verknüpfungsereignissen bereits erfasst worden sind. In einer Konfigurationsphase ist es z.B. konfigurierbar, wie viele Wiederholungen eines Verknüpfungsereignisses als zulässig erachtet werden. So kann es in einigen Anwendungen z.B. erlaubt werden, dass ein Satz von Disposables für einen bestimmten Patienten von ihm mehr als einmal verwendet werden darf. Allerdings kann eine konfigurierbare maximale Anzahl von Wiederholungen definiert worden sein, um eine darüber hinaus gehende Verwendung zu beschränken. Der große Vorteil ist darin zu sehen, dass aufgrund der verteilten Speicherstruktur gewährleistet werden kann, dass der Patient ein initial mit ihm verknüpftes/ihm eindeutig zugewiesenes Disposable mit unterschiedlichen Dialysemaschinen verwenden kann.

Bei Wahl von geeigneten Verknüpfungsfunktionen und/oder Rechenvorschriften ist das Verfahren und System nicht oder nur durch sehr lange dauernde, aufwändige Rechenverfahren korrumpierbar.

Kurze Übersicht über die Figuren

In der folgenden detaillierten Figurenbeschreibung werden nicht einschränkend zu verstehende Ausführungsbeispiele mit deren Merkmalen und weiteren Vorteilen anhand der Figuren der Zeichnung erläutert. In dieser zeigen:

Fig. 1   eine schematische Übersicht über eine verteilte Speicherstruktur gemäß einer ersten Ausführungsform der Erfindung mit mehreren Dialysegeräten, an denen unterschiedliche Patienten behandelt werden können;

Fig. 2   eine schematische Übersicht über eine verteilte Speicherstruktur gemäß einer zweiten Ausführungsform der Erfindung mit mehreren Umkehrosmoseanlagen, die mit unterschiedlichen Membranen betrieben werden können;

Fig. 3   eine schematische Darstellung eines Formats zur Speicherung von Header und Footer in der verteilten Speicherstruktur der Geräte;

Fig. 4   eine schematische Darstellung eine Datenblocks;

Fig. 5   eine alternative Form des in Fig. 4 dargestellten Datenblocks;

Fig. 6   eine schematische Strukturdarstellung von zwei unterschiedlichen Ausführungsbeispielen in einer Gesamtschau mit den jeweiligen Verfahrensschritten und

Fig. 7   ein Ablaufdiagramm eines Verfahrens gemäß einer bevorzugten Ausführungsform der Erfindung.

Detaillierte Beschreibung von Ausführungsbeispielen und der Figuren

Die Erfindung dient dazu, die Sicherheit des Gerätebetriebs von Dialysegeräten DG, die mit Verbrauchsgegenständen, wie Einwegartikeln (z.B. Dialysatoren) betrieben werden müssen, oder anderen medizinischen Geräten, wie z.B. Umkehrosmoseanlagen, die mit Membranen betrieben werden, zu erhöhen.

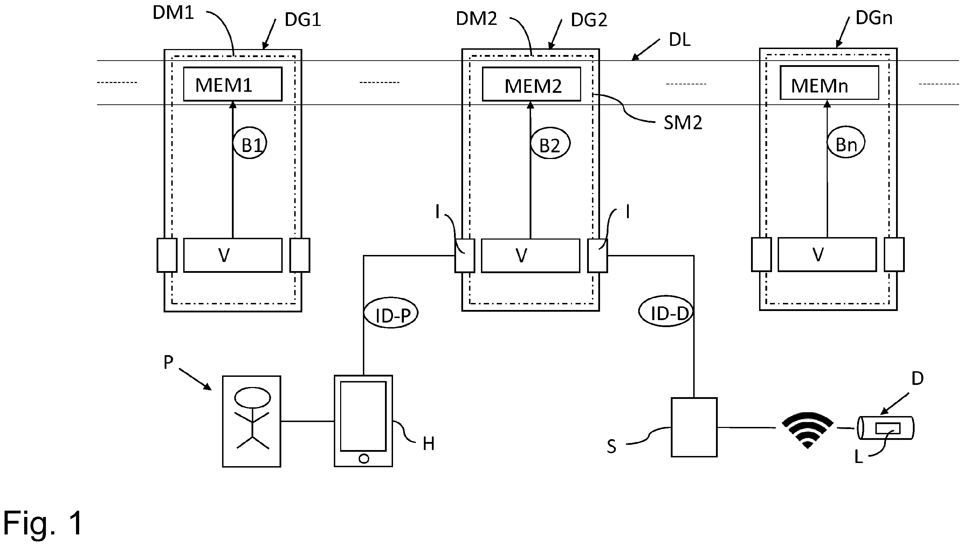

In einer ersten Ausführungsform der Erfindung, die schematisch in Fig. 1 dargestellt ist, bezieht sich ein erster Verknüpfungspartner auf einen Patienten P, der über einen Code oder Identifikator eines Identifikationsmittels, wie z.B. eine Patienten-Karte, eindeutig identifizierbar ist und sich mit seinem Identifikator ID-P an dem Dialysegerät DG anmeldet. Ein für die Behandlung des Patienten vorgesehenes Dialyseset - das Disposable D - stellt einen zweiten Verknüpfungspartner dar. Hintergrund dieser Ausführungsform ist, dass der Gebrauchsgegenstand, z.B. in Form eines Dialysators/ Schlauchsets, dem Patienten zur eigenverantwortlichen Verwendung zur Verfügung gestellt wird und sich der Patient dann mit diesem Set an einer beliebigen Dialysemaschine DG seiner Wahl anmeldet, um die Behandlung durchzuführen. Je nach Anwendungsfall soll der Gebrauchsgegenstand nur einmalig oder nur mit einer begrenzten Anzahl von Wiederholungen, aber auf jeden Fall nur für ein und denselben Patienten verwendet werden, beispielsweise um eine Übertragung von Krankheitserregern zwischen zwei Patienten zu verhindern, die u. U. auch durch eine Reinigung oder Desinfektion des begrenzt wiederverwendbaren Disposables D nicht vollständig beseitigt werden. Die Erfindung löst das Problem, indem ein Dokumentationsverfahren bereitgestellt wird, das so erweitert ist, dass Wiederholungen von Verknüpfungsereignissen fälschungssicher und zudem anonym dokumentiert und/oder überprüft werden können und, dass überprüft werden kann, ob ein bestimmter Verknüpfungspartner bereits ein Verknüpfungsereignis erfahren hat. Zudem kann sichergestellt werden, dass bereits verknüpfte Partner nicht anderweitig verknüpft werden können. Aus Gründen der Infektionsgefahr darf der Gebrauchsgegenstand keinesfalls von einem Patienten zu einem anderen wechseln. Die Erfindung schafft ein System, mit dem dies automatisch sichergestellt werden kann. Dies wird insbesondere gewährleistet durch ein Gerät im medizintechnischen Kontext, in dem das Disposable verwendet werden soll. Das Gerät prüft selbstständig und automatisch die Zulässigkeit einer Verknüpfung der Verknüpfungspartner und kann ggf. eine Behandlung unterbinden oder einschränken.

Wie in Fig. 1 dargestellt, ist auf dem Dialysegerät DG ein Dokumentationsmodul DM ausgebildet. Dieses umfasst eine elektronische Verarbeitungseinheit V, die zur Verarbeitung der beiden Identifikatoren ID-P, ID-D bestimmt ist, die über entsprechende Schnittstellen I eingelesen werden. So kann das Disposable D mit einem Label L gekennzeichnet sein, das mittels eines Scanners S ausgelesen und in Form des zweiten Identifikators ID-D der Verarbeitungseinheit zur Verfügung gestellt wird. Entsprechend kann der erste Identifikator des Patienten ID-P über unterschiedliche technische Optionen eingelesen werden, so z.B. über eine App, die auf dem Mobilfunktelefon H des Patienten installiert ist und über die die Patienten-Nummer an die Schnittstelle I übertragen wird. Auf den ersten Identifikator ID-P wird die ersten Rechenvorschrift RV1 angewendet, um den ersten Wert W1 zu berechnen. Ebenso wird aus dem zweiten Identifikator ID-D die zweite Rechenvorschrift RV2 angewendet, um den zweiten Wert W2 zu berechnen. Beide Werte werden in vordefinierte Bereiche 1.B, 2.B in den Datenblock B geschrieben, der im lokalen Speicher MEM und ggf. synchronisiert in die verteilte Datenstruktur DL gespeichert wird.

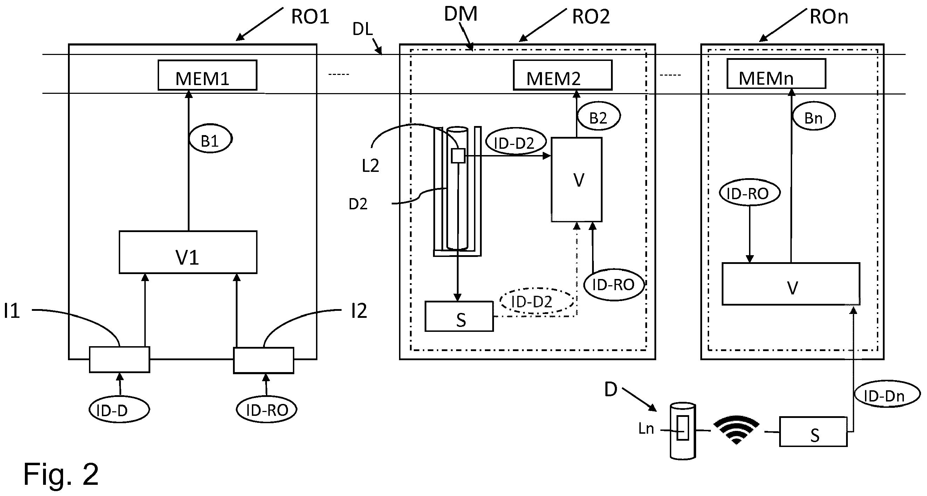

In einer zweiten Ausführungsform der Erfindung, die in **Fig. 2** dargestellt ist, bezieht sich der erste Verknüpfungspartner auf eine Membran D als Gebrauchsgegenstand, die in einer Umkehrosmoseanlage (zweiter Verknüpfungspartner) betrieben werden soll. Die Membran ist gekennzeichnet und z.B. mit einer elektronischen Kennzeichnung L über einen RFID-Chip eindeutig identifizierbar. Die Kennzeichnung L der Membran kann von einem z.B. auf der RO-Anlage ausgebildeten Lesegerät S ausgelesen und in Form eines digitalen zweiten Identifikators ID-D zur weiteren Verarbeitung der Verarbeitungseinheit V bereitgestellt werden. Die Membran D meldet sich sozusagen mit diesem Identifikator ID-D an der Umkehrosmoseanlage RO an. Bei dieser Ausführungsform gilt es zu überwachen, dass die Umkehrosmoseanlage RO stets auf zulässige Weise betrieben wird und die Verbrauchsgegenstände D (z.B. Membran) dabei nicht über den vorgesehenen Verwendungszeitraum hinaus verwendet werden. Das hier vorgeschlagene Dokumentationsverfahren ist deshalb derart erweitert, dass eine bestimmte Anzahl von identischen Verknüpfungsereignissen ermöglicht werden kann, aber alle weiteren Verknüpfungsereignisse zu verhindern. Wenn z.B. die osmotische Membran D länger als vorgesehen eingesetzt wird, kann die Wasserqualität nicht mehr gewährleistet werden und die Dialysemaschine DG, die über die Umkehrosmoseanlage RO versorgt wird, kann nicht mehr gemäß den Sicherheitsstandards betrieben werden. Die osmotische Membran, die in der Umkehrosmoseanlage nur die Trägerflüssigkeit (Solvent) durchlässt und die gelösten Stoffe (Solute) zurückhält, muss diesen hohen Drücken standhalten können. Wenn der Druckunterschied das osmotische Gefälle mehr als ausgleicht, passen die Solventmoleküle wie bei einem Filter durch die Membran, während die "Verunreinigungsmoleküle" zurückgehalten werden. Die Membrane D sind daher sehr sensibel. Um Beschädigungen der Membran zu verhindern, können Filter vorgeschaltet werden. Ein Feinfilter kann mechanische Verunreinigungen, ein Aktivkohlefilter chemische Beschädigungen (z. B. durch Chlor) verhindern. Wie in Fig. 2 dargestellt, gib es unterschiedliche technische Maßnahmen, wie die Identifikatoren ID-D, ID-RO der Verarbeitungseinheit V des Dokumentationsmoduls DM zugänglich gemacht werden können. Diese sind in Fig. 2 an den drei beispielhaft dargestellten Anlagen RO repräsentiert. Bei der links dargestellten ersten Anlage RO1 sind zwei Schnittstellen I1 und I2 bereitgestellt, die zum Einlesen der beiden Identifikatoren ID-RO und ID-D dienen. Dabei kann es sich z.B. auch um Scanner handeln, die zumindest zeitweise dem Gerät RO zugeschaltet sind und Labels bzw. Kennzeichnungen der Anlage selbst und der Membran D einlesen. Alternativ können - wie bei der Anlage RO2 dargestellt - die Einleseprozesse auch alle oder teilweise intern also innerhalb der Anlage RO implementiert sein. Dann ist die Membran D mit einem Label L gekennzeichnet (z.B. ein aufgebrachter Code oder ein in das Material während der Herstellung eingearbeitetes Label), das von einem Scanner der Anlage RO erfasst und als Identifikator ID-D der Verarbeitungseinheit V zugeführt wird. Entsprechend kann die Anlage RO ihren Identifikator ID-RO über einen Speicherzugriff selbst auslesen und der Verarbeitungseinheit V zuführen. In einer weiteren Alternative, die bei der dritten Anlage RO3 dargestellt ist, kann ein Scanner S extern und gegebenenfalls zeitweise bereitgestellt werden, um das Lable Ln der Membran Dn zu scannen und über eine entsprechende Netzwerkverbindung und die Schnittstelle I der Verarbeitungseinheit V der Anlage Ron zuzuleiten. Der Identifikator ID-RO der Anlage selbst kann direkt aus einem Speicherbereich der Anlage eingelesen werden und benötigt keinen externen Scanner S.

Andere Ausführungsformen richten sich auf andere medizintechnische Geräte, die mit anderen Verbrauchsgegenständen betrieben werden und deren Verbrauch und Verwendung im Gerät mit den hier vorgeschlagenen Verfahren manipulationssicher und ohne zentrale Administrationsstrukturen dokumentiert und überwacht werden soll.

Grundsätzlich zielt die hier vorgeschlagene Lösung darauf ab, ein Ereignis zu dokumentieren, das eine Verknüpfung von zwei Verknüpfungspartnern (z.B. Gebrauchsgegenstand/Disposable und Patient, der bei seiner Dialysebehandlung das Disposable verwendet) darstellt. Konkret kann ein solches Verknüpfungsereignis beispielsweise den Fall repräsentieren, dass eine Person einen Gebrauchsgegenstand, etwa einen Dialysator, bei einer Behandlung benutzt (Verbrauchsgegenstände werden in dem Zusammenhang auch "Disposable" genannt.).

In einer Weiterbildung kann nicht nur der Betrieb des Gerätes dokumentiert werden, sondern das Gerät kann auch gegen eine unautorisierte Verwendung von Verbrauchsgegenständen auf dem Gerät abgesichert werden. Wenn ein intendiertes Verknüpfungsereignis von zwei Verknüpfungspartnern als nicht autorisiert ausgewertet worden ist, dann kann das Gerät zumindest für genau diese Verwendung mit den beiden Verknüpfungspartnern gesperrt werden. In Notfällen ist jedoch eine manuelle Sondergenehmigung möglich, indem ein manuelles Überschreiben erfolgt. Dazu kann die Eingabe eines vordefinierten Freigabecodes auf dem Gerät notwendig sein.

Es wird vorausgesetzt, dass die jeweiligen Verknüpfungspartner über eine/n ihnen jeweils zugeordnete/n Kennzeichnung bzw. einen Identifikator eindeutig identifiziert werden können. So kann sich der Patient z.B. über seine Patientenkarte, und der vom Patienten mitgeführte Dialysator über einen unlösbar damit verbundenen RFID-Chip identifizieren. Ebenso kann die Membran über einen unlösbar daran angebrachten RFID-Chip identifizierbar sein.

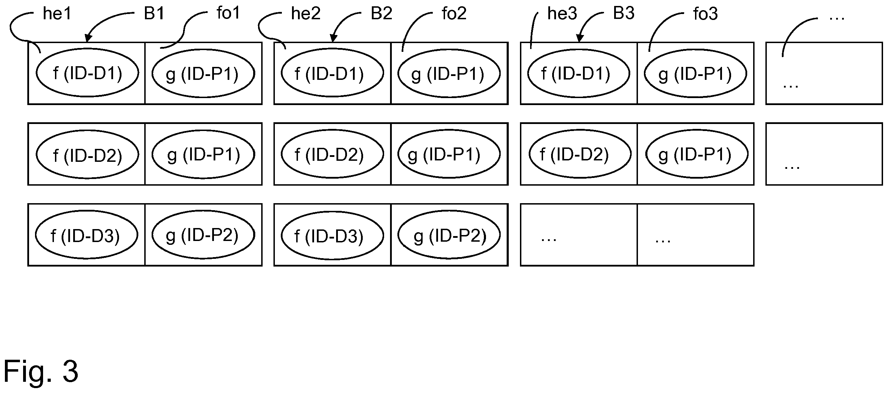

In Zusammenhang mit **Fig. 3** wird wie folgt beschrieben, wie ein Verknüpfungsereignis dokumentiert wird:

> a) eine erste mathematische Rechenvorschrift RV1, z.B. Funktion g, auf den ersten Identifikator ID-P/RO angewendet wird: g(ID-P/RO)
> b) eine zweite mathematische Rechenvorschrift RV2, z.B. eine mathematische Funktion f, auf den zweiten Identifikator (z.B. ID-D) angewendet wird: f(ID-D)
> c) ein Datenblock wird geschrieben, indem f(ID-D) in den einen ersten Datenbereich 1.B, den Header und g(ID-P/RO) in einen zweiten Datenbereich 2.B, den Footer, geschrieben wird.
> d) Der Datenblock B wird in einem Speicher MEM, DL geschrieben.

Wie in Fig. 3 ersichtlich, werden so Ketten von mehrere Blöcken, also ein Mehr-Ketten-Raum aufgebaut, wobei eine Kette jeweils Verknüpfungsereignisse von einem Verknüpfungspaar (D-P oder D-RO) repräsentiert. Die Abfolge der Blöcke kennzeichnet somit die Wiederholungen der Verknüpfungen bzw. des Gerätebetriebs (z.B. von Behandlungen). Das erste Disposable D1 ist in der ersten Kette dem ersten Patienten P1 zugeordnet. Ein zweites Disposable D2 ist demselben Patienten P1 zugeordnet (er wurde also mit einem "neuen" Disposable behandelt). In der dritten Kette wird das dritte Disposable D3 einem zweiten Patienten P2 zugeordnet.

Die erste und die zweite mathematische Funktion können identisch sein.

Im einfachsten Fall ist f eine Multiplikation mit 1, dann ist f(ID-D) = ID-D. In einem bevorzugten Fall ist f eine kryptologische Funktion oder eine Hash-Funktion (auch Streuwertfunktion), wie z.B. SHA, SHA-1, MD5. Andere Funktionen sind vorstellbar.

Dasselbe gilt analog für g.

In einem bevorzugten Ausführungsbeispiel sind f, g Hashfunktionen, so dass der jeweils erzeugte Wert eine fest definierte Länge hat und quasi einen Fingerabdruck der Identifikatoren repräsentiert. Der dann entstehende Dokumentationswert W = f(ID-D) φ g(ID-P) φ k ist kryptologisch wertvoll und nicht einfach vorherzusehen oder auf die ausgangswerte ID-D und ID-P zurückzuführen. Dies hat den Vorteil, dass die tatsächliche ID (ID-D oder ID-P) in dem Datensatz nicht im Klartext erkennbar wäre, was erhöhten Ansprüchen an die Wahrung der Privatsphäre entgegenkommt und auch die Fälschung von Seriennummern der Disposables erschwert.

In einem bevorzugten Ausführungsbeispiel sind die Identifikatoren ID-D und ID-P einzigartig und in einer schwer oder nicht vorhersehbaren Weise erzeugt, z.B. unter Verwendung eines Zufallsgenerators.

Wenn man nun eine Vielzahl von Disposables D_1 bis D_1000 hat, haben all diese Disposables unterschiedliche, nicht zu erratende oder vorhersagbare, eindeutige Identifikatoren ID-D_1 bis ID-D_1000.

Wenn man nun außerdem eine Vielzahl von Personen P_1 bis P_1000 unterschiedliche, nicht zu erratende oder vorhersagbare, eindeutige Identifikatoren ID-P_1 bis ID-P_1000 zuordnet, ist man in der Ausgangslage für das letztgenannte bevorzugte Ausführungsbeispiel.

Für jede beliebige Kombination von Verknüpfungspartnern (D_i, P_j) entsteht mittels dieser Idee ein eindeutiger, kryptologisch sicherer, nicht vorhersehbarer Wert. Durch die Auswahl geeigneter kryptologischer Funktionen für f, g und ggf. φ kann ein Rückrechnen auf die Ursprungswerte beliebig schwierig bis praktisch unmöglich gemacht werden. Die berechneten Dokumentationswerte W1, W2 stellen also ein eindeutiges Muster oder eine eindeutige Signatur der bei dem Verknüpfungsereignis beteiligten Verknüpfungspartner dar.

Ein Angreifer, der einen Identifikator nicht kennt, wird viel Aufwand betreiben müssen, um eine sinnvolle Kombination zu erraten, da die Identifikatoren nicht leicht erratbar sind. Ein Gerät, das lokal die Identifikatoren zweier Verknüpfungspartner einlesen kann, hat es dagegen leicht. Dies hat den technischen Vorteil, dass ein Lesezugriff auf eine verteilte Speicherstruktur DL, sehr effizient ausgeführt werden kann.

In dem hier vorgestellten Vorschlag geht es in erster Linie um ein Verfahren und eine Datenstruktur zur Verknüpfung von kryptografischen ersten und zweiten Werten aus zwei Identifikatoren in verschiedenen Datenbereichen eines Datenblocks B . In dem Datenblock B wird eine Verknüpfung in einer Datenstruktur dokumentiert, in der die Identifikatoren der beiden Verknüpfungspartner an verschiedenen Stellen eingehen.

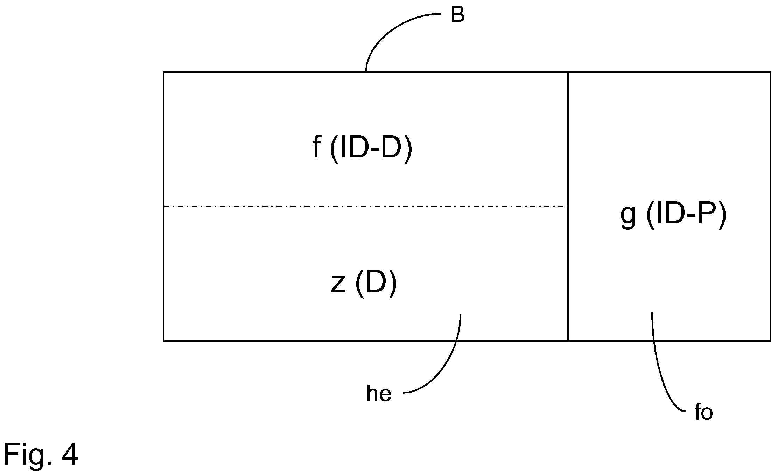

Es wird vorausgesetzt, dass es zwei Identifikatoren ID-P/ID-RO sowie ID-D gibt, über die der jeweilige Verknüpfungspartner P/RO, D eindeutig identifiziert werden kann. Dadurch ergibt sich eine Datenstruktur, die einen wohl-definierten bestimmten Aufbau hat, der ein effizientes Verarbeiten in der Datenstruktur (z.B. Zur Abfrage eines der beiden Werte) ermöglicht. In einer ersten Variante hat ein Datenblock B eine Datenstruktur, die aus einem ersten Bereich 1.B und einem zweiten Bereich 2.B besteht. In **Fig. 4** ist schematisch dargestellt, dass ein erster Block B1 linksseitig einen Header, in den Figuren mit Bezugzeichen he gekennzeichnet und rechtsseitig gezeigt, einen Footer, in den Figuren mit Bezugszeichen fo gekennzeichnet, umfasst.

In Weiterbildungen des Verfahrens kann der Header h erweitert sein (z.B. um einen Zählwert z(D)) der wiederholte Verwendungen insbesondere eines Gebrauchsgegenstandes repräsentiert. Dies ist schematisch in Fig. 4 dargestellt. Der Block B umfasst in diesem Ausführungsbeispiel einen Header he und einen Footer fo, wobei der Header um den Zählwert z(D) erweitert sein kann, um Wiederholungen des Verknüpfungspaares effizient zu dokumentieren.

Ein Header mit einer Zählwert-Abhängigkeit oder Datums-Abhängigkeit ist nicht zwingend erforderlich. Eine Zählung von Blöcken im Ledger wäre auch anderweitig möglich, im einfachsten Fall tatsächlich durch primitives Abzählen. Allerdings erscheint es am praktikabelsten, einen um eine von einem Zählwert abhängige Erweiterung in den Header einzubringen.

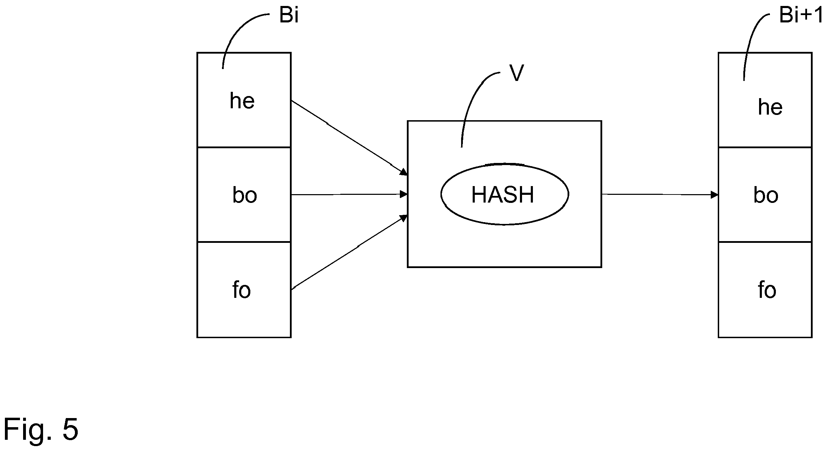

In einer anderen vorteilhaften Ausführungsform der Erfindung hat der Datenblock B eine andere Datenstruktur, die drei Bereiche umfasst: einen Header he, einen Footer fo und einen Body bo. Wie in **Fig. 5** dargestellt, kann der Body bo einen Verweis auf vorhergehenden Datenblöcke B umfassen. Der Verweis kann verschlüsselt, z.B. verhasht sein. Des Weiteren kann der Body um eine Verknüpfung der Identifikatoren oder Blöcke miteinander erweitert sein. Weiterhin ist eine Erweiterung des Bodys um weitere Parameter der dokumentierten Verknüpfung bzw. Transaktion möglich.

Dabei bleibt aber unberührt, dass der von dem zweiten Identifikator ID-D abhängige Anteil im Header konstant bleibt. Damit können alle Blöcke mit derselben f(ID-D) im Header leicht in einem globalen Ledger mit vielen Blöcken B aufgefunden werden. Das gilt entsprechend für den zweiten Identifikator im Body / Footer. Die Datenstruktur bleibt somit konstant, was Verarbeitungen und spätere Prozesse erleichtert.

Zur Erhöhung der Sicherheit und zur Gewährleistung einer Block-übergreifenden Datensicherheit und Daten-Integrität kann in Weiterbildungen des Grundprinzips zusätzlich eine Verknüpfung oder Hashfunktion davon in den Body eines Folgeblocks geschrieben werden, wie dies beispielsweise in Fig. 5 schematisch dargestellt ist. Hierbei enthält der Block B(n) zur Wiederholung n der Verknüpfung von ID-D und ID-P also folgende Bestandteile:

    1. Header he: f(ID-D), h (z(D));
    2. Body bo, der optional ist: Verknüpfung von mehreren Werten aus vorherigen Block (z.B. Anwendung einer kryptographischen Funktion auf die verknüpften Werte oder Verknüpfung mehrerer Werte durch eine kryptographische Funktion, z.B. Hash seeding oder hash superseeding), weitere Parameter.

    3. Footer fo: g(ID-P)

Die Blöcke B werden nach ihrer Berechnung auf eine Weise gespeichert und verteilt, die ermöglicht, dass mehrere Geräte, die verteilt sind und an diesem Verfahren beteiligt sind, darauf zugreifen, d.h. speichern und lesen können. Die übergeordnete, gemeinsame Datenstruktur, in der alle Blöcke des Verfahrens gespeichert werden, ist vorzugsweise als distributed Ledger DL eingerichtet.

In einer bevorzugten Variante sind die Eigenschaften der Identifikatoren ID-D, ID-P/ ID-RO und der Funktionen f, g, h so gewählt, dass alle mit dem Verfahren erzeugbaren Werte eine gleichbleibende Größe und/oder ein gleichbleibendes Format haben. Dadurch ergibt sich eine Speicherung mit gleichgroßen Blöcken. Gegebenenfalls kann ein Padding mit Nullen oder Einsen auf die einheitliche Blockgröße erforderlich sein. Die Speicherung kann lokal in einem Endgerät stattfinden, das das Verfahren ausführt. Die Speicherung kann remote in der Cloud, auf einem Server, in einem Backend stattfinden. Dann wird aber ein lokales Gateway-Gerät benötigt, das wiederum in Datenaustausch mit den Geräten RO, DG steht. Bevorzugt findet die Speicherung so statt, dass alle beteiligten Geräte die Muster direkt nach der Erzeugung lokal speichern und danach an im Netzwerk verbundene Geräte verteilen. Bevorzugt gibt es eine Vielzahl von in einem Netzwerk verbundenen Geräten, die das Verfahren ausführen und z.B. über das Internet mithilfe z.B. einer Peer-to-Peer- Kommunikation inkrementell ihre Speicher so synchronisieren, dass nach einer gewissen Zeit alle erzeugten Blöcke im lokalen Speicher MEM aller Geräte vorliegen. Ein derart aufgestelltes Netzwerk benötigt keine zentralen Instanzen. Alternativ kann die Verteilung der Blöcke auf die im Netzwerk verbundenen Geräte DG, RO mittels eines Algorithmus so gesteuert sein, dass nicht jedes Gerät alle Muster speichert, aber alle Muster innerhalb einer definierten Gruppe von kommunikativ verbundenen Geräten zumindest einmal, vorzugsweise redundant gespeichert sind. Die Verteilung der Blöcke B zur Speicherung kann ohne dedizierten zentralen Verwaltungs-Master nach grundsätzlich bekannten Verfahren erfolgen. Dabei kann vorzugsweise eine Speicherung wiederholt in Zusammenhang mit einem Gerät verwendeter Identifikatoren lokal in diesem Gerät erfolgen, um den Netzwerkverkehr zu reduzieren. Bevorzug wird dabei eine Distributed-Ledger-Technologie verwendet, um Übereinkunft über alle durch die Blöcke B dokumentierten Verknüpfungsereignisse innerhalb des Gerätenetzwerks zu erreichen und diese Blöcke B im Netzwerk zu aktualisieren. So ergibt sich eine dezentrale Verknüpfungsereignisdatenbank. Optional wird ein Konsens-Mechanismus zur Gewährleistung identischer Kopien des distributed Ledger auf allen beteiligten Instanzen angewendet.

Wenn man ein Verknüpfungsereignis (wie einen Gerätebetrieb z.B. zur Dialysebehandlung) herbeiführen möchte und die Identifikatoren ID-D, ID-P/ID-RO beider Verknüpfungspartner D, P/RO kennt kann man mit einem Gerät das Verfahren ausführen und erhält einen Block B zu diesen Partnern. Nun kann der distributed Ledger durchsucht werden, ob darin irgendwo ein Block B mit einem Header mit f(ID-D) gespeichert ist. Wenn man keinen Block mit einem Header mit f(ID-D) auffindet, bedeutet das, dass noch kein Verknüpfungsereignis für den Verknüpfungspartner (Disposable) mit der Funktion f(ID-D) dokumentiert ist. Wenn nur eine einzige, festgelegte Funktion f verwendet wird, dann bedeutet das zugleich, dass noch kein Verknüpfungsereignis dieser beiden Verknüpfungspartner dokumentiert ist. Wenn man einen Block mit f(ID-D) im Header auffindet, bedeutet das, dass ein Verknüpfungsereignis von ID-D (und f(ID-D)) gegeben hat. Der Footer des aufgefundenen Blocks gibt darüber Aufschluss, mit welchem Partner P die Verknüpfung stattfand. Wenn das ausführende Gerät die Funktion g kennt und die ID-P kennt, kann es g(ID-P) erzeugen und mit dem Footer vergleichen. Wenn die beiden Werte identisch sind und ID-P eine einzigartige ID ist sowie nur eine festgelegte Funktion (oder ein Satz von unterscheidbaren, festgelegten Funktionen g) verwendet wird, ist sichergestellt, dass es eine Verknüpfung dieser beiden Verknüpfungspartner D und P gegeben hat. In einer Variante kann das dazu führen, dass kein weiteres Verknüpfungsereignis zugelassen wird, d.h. nur eine einzige Verknüpfung pro Paar wird zugelassen.

Die Geräte müssen folgende Minimalanforderungen erfüllen, um ein erfindungsgemäßes Verfahren ausführen zu können:

- Sie müssen in Kenntnis von ID-D gelangen können (durch manuelle Eingabe, Scanner für Barcode oder QR-Code, RFID-Leser, Bildererkennung, Datenempfang);
- Sie müssen in Kenntnis von ID-P gelangen können (durch einen Patientenkartenleser und/oder den oben beschriebenen Methoden bzw. von ID-RO;
- Sie müssen eine Kommunikationsverbindung haben;
- Sie müssen einen Speicher haben; vorzugsweise eine lokale Speicherstruktur und eine verteilte Speicherstruktur, die Bestandteil der distributed ledger ist;
- Die kryptografischen Berechnungen können lokal oder remote erfolgen. Wenn sie lokal erfolgen sollen, muss das Gerät natürlich in der Lage zur Berechnung sein, d.h. die Berechnungsvorschriften f, g, h kennen, die Zählungs- oder Datumsoperation ausführen können, um z(D) zu bestimmen, und ausreichend Prozessor- und Speicherressourcen aufweisen;
- Die Internetanbindung kann direkt oder über Zwischeninstanzen (wie lokales WiFi) erfolgen.

Konkrete Beispiele für die Umsetzung der hier vorgeschlagenen Lösung sind Behandlungsmaschinen, wie Dialysegeräte, und medizinische Anlagen (z.B. RO-Anlagen), die mit Verbrauchsgegenständen, wie Membranen, betrieben werden. Die genannten Geräte können entweder selbst Teilnehmer des Dokumentationssystems sein oder sie können mit Mittlerknoten bzw. Gateways interagieren, z.B. Smartphones bzw. Tablets, die eine Behandlung dokumentieren und/oder autorisieren/sperren/freigeben können. In diesem Fall sind die Gateways die Teilnehmer an dem System.

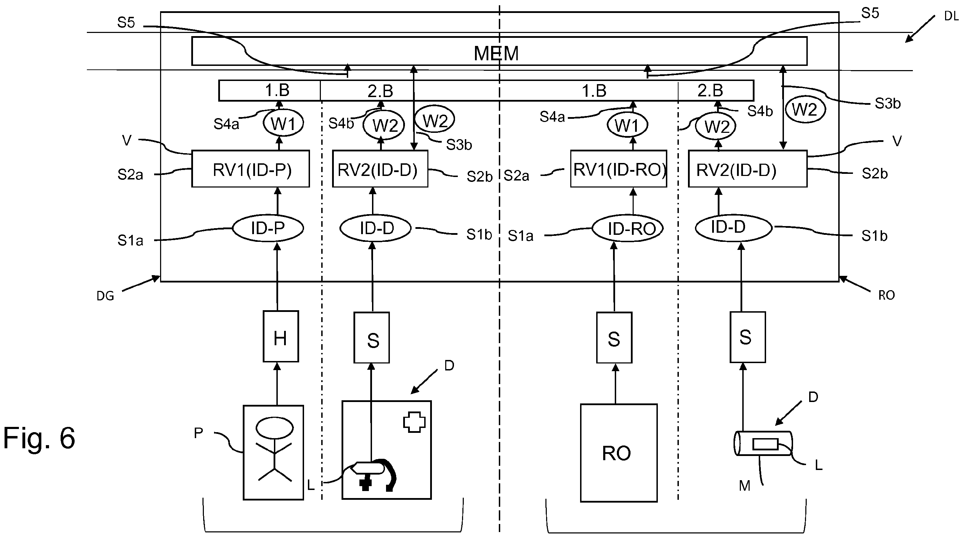

Fig. 6 zeigt die beiden genannten Ausführungsbeispiele in einer Figur. Die gestrichelte Linie trennt die beiden Ausführungsbeispiele. Links ist das Beispiel dargestellt: Patient P wird an einem Dialysegerät DG mit einem Disposable D (z.B. Schlauchset) behandelt. Rechts ist das Beispiel dargestellt: eine RO-Anlage RO wird mit einem Membran D als Disposable betrieben. Die Disposables D sind mit einem Label L gekennzeichnet, der von einem Scanner S oder von einem anderen Gerät H erfasst wird und in digitaler Form als Identifikator ID-P/ID-RP, ID-D in Schritt S1a, S1b einer Verarbeitungseinheit V zugeführt werden (über die Schnittstelle, die in Fig. 6 nicht dargestellt ist). In der Verarbeitungseinheit V werden daraufhin in Schritt S2a, S2b die genannten Rechenvorschriften RV1, RV2 angewendet werden. Optional kann eine Validierung in Schritt S3b durchgeführt werden. Nach erfolgreicher Validierung, die in Schritt S3b unter Zugriff auf die verteilte Datenstruktur DL erfolgt, kann der Block B in Schritt S4a, S4b in eine Speicherstruktur MEM, DL der verteilten Datenstruktur geschrieben werden. Der berechnete erste Wert W1 wird in Schritt S4a in den ersten Bereich 1.B geschrieben und der berechnete zweite Wert W2 wird in Schritt S4b in den zweiten Bereich 2.B des Datenblocks B geschrieben. Dabei kann der Block B in einer lokalen Speicherstruktur des Gerätes gespeichert werden.

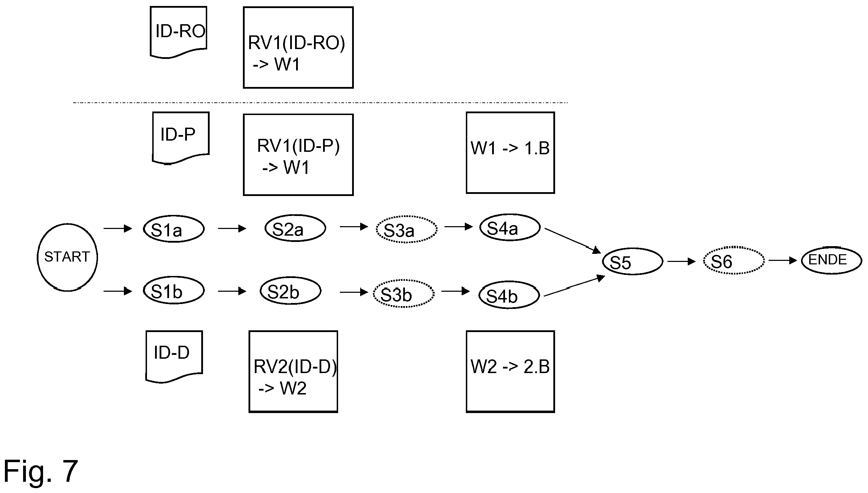

Fig. 7 zeigt ein Ablaufdiagramm wieder in einer gemeinsamen Darstellung für unterschiedliche Ausführungsbeispiele. Die Verfahrensschritte sind mittig dargestellt und oben ist der geräteseitige Verknüpfungspartner P, RO gezeigt und unten ist der Verknüpfungspartner gezeigt, der ein Gebrauchsgegenstand D ist. Nach dem Start des Verfahrens, werden in Schritt S1a, S1b die Identifikatoren ID-P/ID-RO, ID-D der Verknüpfungspartner eingelesen. In den Schritte S2a, S2b werden die Rechenvorschriften RV1, RV2 angewendet, um den ersten Wert W1 und den zweiten Wert W2 zu berechnen und in Schritt S4a, S4b in den ersten bzw. zweiten Bereich 1.B, 2.B des Blockes zu schreiben. Optional kann in Schritt S3b eine Validierung des berechneten zweiten Wertes W2, der das Disposable D repräsentiert, ausgeführt werden. Die Validierung wird unter Zugriff auf die verteilte Speicherstruktur DL oder auf den lokalen Speicher MEM ausgeführt. Bei erfolgreicher Validierung kann der Block B mit dem ersten und zweiten Wert W1, W2 auf synchronisierte Weise in Schritt S5 in die verteilte Datenstruktur DL geschrieben werden. Falls keine Validierung erfolgt, wird der Block B mit den Werten W1, W2 in Schritt S5 direkt und auf synchronisierte Weise in die verteilte Datenstruktur DL und/oder in den Speicher MEM geschrieben. In Schritt S6 kann optional eine Autorisierung ausgeführt werden, in der weitere Prüfkriterien zur intendierten Verwendung des Disposables D auf Einhaltung überwacht werden. In Fig. 7 sind die optionalen Schritte mit gepunkteter Linie gekennzeichnet.

Üblicherweise wird die Validierung nur für den zweiten Wert W2, der das Dispoable D repräsentiert, ausgeführt, um zu validieren, ob das Disposable D nicht schon einmal verwendet worden ist. In anderen Anwendungsfällen ist es allerdings auch sinnvoll, die Validierung nicht nur Gebrauchsgegenstandsseitig sondern auch für den anderen Verknüpfungspartner durchzuführen. Dann wird ebenfalls die in Schritt S3a dargestellt Validierung ausgeführt.

Mit dem grundlegenden Verfahren sehen die Blöcke B von zwei Verknüpfungsereignissen derselben Verknüpfungspartner gleich aus, denn für beide Ereignisse würde der Header zunächst f(ID-D) aufweisen und der Footer g(ID-P). Zur Unterscheidung wurde daher weiter oben bereits erwähnt, dass eine Funktion h von einem Zählwert oder einem Datum oder ein anderweitig gearteter Unterscheidungswert z(D) in den Header aufgenommen werden soll. Dadurch sind Blöcke derselben Partner aber unterschiedlicher Wiederholungen / Daten über ihre Header bereits unterscheidbar. Es ist auch denkbar, dass es z.B. eine Tabelle gibt, die jedem z einen Identifikator ID-z zuordnet. Hierbei ist wichtig, dass alle an dem Verfahren beteiligten Instanzen auf die gleiche Weise einem Wiederholungswert z einen Identifikator IDz zuordnen, so dass die Struktur einheitlich ist und alle Abfragemechanismen auf derselben Grundlage erfolgen. Damit ist es möglich, dass sich alle beteiligten Geräte und Instanzen ohne Eingriff von außen (ohne einen zentralen Administratorknoten) von dem Wert einer Wiederholung zum nächsten "hangeln" können.

Bei Wahl von geeigneten Werten für z(D) kann so sichergestellt werden, dass die Werte W zu verschiedenen Wiederholungen (d.h. mit verschiedenen Wiederholungszahlen z) verschieden sind. Wenn ein Gerät nicht weiß, wie viele Wiederholungen durchgeführt wurden, und das ermitteln möchte, kann es nacheinander verschiedene Blöcke B zu den vorliegenden Verknüpfungspartnern ausrechnen und den distributed ledger DL nach den Blöcken durchsuchen. Der bei der Durchsuchung gefundene Block B zu der höchsten Wiederholungszahl z entspricht der Anzahl bisher erfolgter und dokumentierter Wiederholungen.

Ein praktischer Fall für die Anwendung einer Weiterbildung dieses Verfahrens ist es, sicherzustellen, dass ein bestimmtes Disposable D (z.B. Dialysator), welches für die mehrmalige Benutzung geeignet und vorgesehen ist, nur von derselben Person P verwendet wird. Mit den oben gezeigten Verfahren kann man eine Verknüpfungsereignis zweier Verknüpfungspartner erzeugen und die Wiederholung eines identischen Ereignisses ermitteln und dokumentieren. Wenn ein Disposable D zunächst mit P_1 verknüpft wird, muss ein auf den praktischen Anwendungsfall anwendbares Verfahren sicherstellen, dass D nicht auch noch zusätzlich mit einer Person P_2 verknüpfbar ist. Wenn eine beliebige Instanz im Netzwerk eine 1D-D scannt und das Verfahren anwendet, wird zuerst f(ID-D) erzeugt und im distributed ledger danach gesucht. Wenn ein Block mit f(ID-D) gefunden wird, ist klar, dass im Footer des Blocks die Signatur g(ID-P) des Verknüpfungspartners P zu finden ist. Alle Blöcke zu D dürfen nur der gleichen 1D-P zugeordnet sein. Nun kennt die Netzwerkinstanz auch ID-P und g und berechnet g(ID-P). Ist es in Übereinstimmung mit dem gefundenen Block, dann sind die Partner die gleichen und das Verfahren kann zugeordnet werden. Wenn der errechnete Wert g(ID-P) nicht zu dem im aufgefundenen Block B gespeicherten Wert g(ID-P) passt und nur identische Funktionen g verwendet werden, sind die beteiligten Verknüpfungspartner P nicht identisch. In unserem Beispiel würde das Gerät also eine Fehlermeldung ausgeben und/oder den Start verweigern und/oder einen 'manual override' zum Start erfordern, der wiederum fälschungssicher dokumentiert wird, und/oder eine Fehlermeldung an einen Backbone schicken und/oder die Schwester rufen oder weitere Prozesse automatisch initiieren. In einer Weiterbildung wird die Verknüpfungslogik umgekehrt und es sind nur solche Verknüpfungen erlaubt, die es noch nicht gegeben hat. Wird kein Block B aufgefunden, der f(ID-D) im Header aufweist, ist noch keine Verknüpfung von D dokumentiert. Damit wäre D mit beliebigen P verknüpfbar und so könnte der erste Block erzeugt werden.

Eine Weiterbildung des Verfahrens ermöglicht, eine bestimmte Anzahl von Wiederholungen z_max zu erlauben und danach (z>z_max) keine weiteren Wiederholungen der Verknüpfungsereignisse mehr zuzulassen. Bei einer einfachen Variante, würden die beteiligten Instanzen eine Maximalanzahl kennen - entweder lokal oder im Gerätenetzwerk oder bei einer Zentralinstanz wäre z_max abgelegt. Dieses Szenario könnte - vor allem bei lokaler Speicherung ein Angriffsrisiko mitbringen. In jedem Fall würden aber auch Wiederholungen über dem Maximum im distributed ledger dokumentiert und es könnte die Haftung ausgeschlossen werden . In einer Weiterbildung ist die Anzahl z_max eine spezifische Eigenschaft des Verknüpfungspartners D und kryptologisch in dessen Identifikator ID-D enthalten, z.B. als Prüfsumme. In einer Weiterbildung ist die Anzahl der Wiederholungen z_max spezifisch für jeden Verknüpfungspartner P festgelegt und in dessen ID-P kodiert. In einer Weiterbildung ist die Anzahl der Wiederholungen sowohl von P als auch von D abhängig und aus einem Raum möglicher Maxima wird in Abhängigkeit von ID-D und ID-P ein Wert bestimmt. In einer Weiterbildung sind die Identifikatoren ID-z der Wiederholungszahl z nur für eine bestimmte Menge bis hoch zu einem ausgewählten z_max definiert, z.B. für z Element aus (1-10) bis z_max=10 oder für z Element aus (0-3) mit z_max=3. Versucht nun eine Instanz, einen Wert W(z>z_max) zu erzeugen, wird sie scheitern, da sie keinen Identifikator ID-z für z>z_max erzeugen kann. Es wird ein Fehler auftreten, der als "Anzahl der maximalen Wiederholungen erreicht" ausgegeben oder dokumentiert werden kann, einen Abbruch oder eine Warnung, dass die Garantie erlischt etc. In einer Weiterbildung wird ID-z mit einer Funktion auf z gebildet, die kein Ergebnis oder kein sinnvolles Ergebnis liefert für Werte von z>z_max. Beispielsweise könnte ID-z eine Funktion t(z) aufweisen, die für z=O bis z=z_max=10 reale Ergebnisse liefert und für natürliche Zahlen z größer 10 imaginäre Ergebnisse liefert:

$$t(z)=\sqrt{(9,99-z)}$$

Alternativ könnte bei der Erzeugung des ersten Blocks B der höchste Zählwert z im Block dokumentiert werden [z.B. durch h(ID-z)]. Dann könnte von der maximal zulässigen Wiederholungszahl z= z_max bei jeder weiteren Verwendung heruntergezählt werden. Bei Erreichen von z=l wäre dann die maximal zulässige Anzahl Wiederverwendungen erreicht und beim nächsten Dekrementieren (z=O) könnte die Funktion ein ungültiges Ergebnis zurückgeben.

Durch eine Weiterbildung des hier vorgestellten Vorschlags, bei der im Body bo eines Blocks B Werte des vorherigen Blocks Bni-1 kryptographisch verknüpft abgelegt sind, lässt sich die Sicherheit deutlich erhöhen und wird für den gewünschten Anwendungsfall ausreichend.

Die erzeugten Datenräume sind leicht zu durchsuchen: Man kann nach Headern suchen, die für jedes Auftreten der ID-D gleichartige Anteile aufweisen. Man kann nach Footern suchen, die für jedes Auftreten der ID-P gleichartige Anteile aufweisen. Damit kann man also Suchergebnisse in Abhängigkeit von D oder von P erhalten und auswerten. Die erhaltenen Datenstrukturen sind für die Verknüpfungspartner aufschlussreich.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Für einen Fachmann ist es insbesondere offensichtlich, dass die Erfindung nicht nur für Dialysegeräte DG und Umkehrosmoseanlagen RO angewendet werden kann, sondern auch für andere medizintechnische Geräte, die Verbrauchsgegenstände verwenden, deren Verwendung an oder mit dem jeweiligen Gerät dokumentiert werden soll. Des Weiteren können die Bauteile des Dokumentationsmoduls DM auch auf mehrere physikalischen Produkte verteilt realisiert werden kann.

Der Schutzbereich der vorliegenden Erfindung ist durch die folgenden Ansprüche gegeben und wird durch die in der Beschreibung erläuterten oder den Figuren gezeigten Merkmale nicht beschränkt.

**Patentansprüche**

1. Verfahren zur manipulationssicheren Dokumentation eines Gerätebetriebs eines Gerätes (DG, RO) aus einer Menge von Geräten, wobei das Gerät (DG, RO) mit zumindest zwei Verknüpfungspartnern betrieben wird und wobei die Geräte (DG, RO) über ein Netzwerk verbunden sind, mit folgenden Verfahrensschritten:

   - Einlesen (S1a) eines ersten Identifikators (ID-P, ID-RO) zur eindeutigen Identifikation eines ersten Verknüpfungspartners (P, RO) und Einlesen (S1b) eines zweiten Identifikators (ID-D) zur eindeutigen Identifikation eines zweiten Verknüpfungspartners (D), der ein Gebrauchsgegenstand (D) zur Verwendung mit dem Gerät (DG, RO) ist;
   - Anwenden (S2a) zumindest einer ersten Rechenvorschrift (RV1) auf den eingelesenen ersten Identifikator (ID-P, ID-RO) zur Berechnung eines ersten Wertes (W1) und
   - Anwenden (S2b) zumindest einer zweiten Rechenvorschrift (RV2) auf den eingelesenen zweiten Identifikator (ID-D) zur Berechnung eines zweiten Wertes (W2);
   - Schreiben (S4a) des berechneten ersten Wertes (W1) in einen ersten Bereich (1.B) eines Datenblocks (B);
   - Schreiben (S4b) des berechneten zweiten Wertes (W2) in einen zweiten Bereich (2.B) des Datenblocks (B);
   - Veranlassen einer synchronisierten Speicherung (S5) des zweiteilig beschriebenen Datenblocks, der ein Verknüpfungsereignis des ersten und zweiten Verknüpfungspartners repräsentiert, in Speichern von Geräten des Netzwerkes.

2. Verfahren nach dem vorangehenden Verfahrensanspruch 1, bei dem das Verfahren zusätzlich - und insbesondere vor dem Schreiben (S4a, S4b) - ein Validieren (S3b) zumindest des berechneten zweiten Wertes (W2) durch Anwendung eines Konsensus-Algorithmus umfasst, so dass das Veranlassen der synchronisierten Speicherung (S5) nur bei erfolgreicher Validierung (S3b) ausgeführt wird.

3. Verfahren nach einem der vorangehenden Verfahrensansprüche, bei dem das Verfahren zur Absicherung des Gerätes vor einem nicht autorisierten Betrieb verwendet wird, indem ein Autorisieren (S6) des Gerätebetriebs für den ersten und zweiten Verknüpfungspartner (P, RO; D) nur dann erfolgt, wenn zumindest der berechnete zweite Wert (W2) erfolgreich validiert (S3b) werden konnten.

4.  Verfahren nach einem der vorangehenden Verfahrensansprüche, bei dem zum Zwecke des Validierens (S3b) zugegriffen wird auf zumindest einen Speicher einer verteilten Speicherstruktur (DL), um für zumindest den berechneten zweiten Wert (W2), der dem Gebrauchsgegenstand (D) zugeordnet ist, zu prüfen, ob dieser bereits gespeichert ist und bei dem nur verneinendenfalls und/oder bei Unterschreiten einer vordefinierbaren Anzahl von zulässigen Wiederholungswerten ein Validieren (S3b) erfolgt.

5.  Verfahren nach einem der vorangehenden Verfahrensansprüche, bei dem das Gerät (DG, RO) für den intendierten Betrieb bei nicht erfolgreicher Validierung (S3b) und/oder bei fehlender Autorisierung (S6) für die jeweiligen Gebrauchsgegenstand (D) gesperrt wird.

6.  Verfahren nach einem der vorangehenden Verfahrensansprüche, bei dem ein Validieren (S3b) und/oder ein Autorisieren (S6) des Verknüpfungsereignisses zeitlich getaktet und/oder ereignisbasiert ausgeführt wird.

7.  Verfahren nach einem der vorangehenden Ansprüche, bei dem das synchronisierte Speichern (S5) unter Verwendung einer verteilten Speicherstruktur erfolgt, insbesondere einer Distributed Ledger Struktur (DL).

8.  Verfahren nach einem der vorangehenden Verfahrensansprüche, bei dem der Datenblock (B) ein festes, vordefiniertes Format hat.

9.  Verfahren nach einem der vorangehenden Verfahrensansprüche, bei dem der Datenblock (B) zweiteilig.

10. Verfahren nach einem der vorangehenden Verfahrensansprüche 1 bis 8, bei dem der Datenblock (B) dreiteilig ist und einen dritten Bereich umfasst, in dem ein Verweis auf vorhergehende Datenblöcke gespeichert ist.

11. Verfahren nach einem der vorangehenden Verfahrensansprüche, bei dem das Verfahren ein Berechnen eines dritten Identifikators als Wiederholungswert umfasst, der eine Anzahl an Wiederholungen für ein Verknüpfungsereignis zwischen dem ersten und zweiten Verknüpfungspartner eindeutig repräsentiert, so dass Wiederholungen von Verknüpfungsereignissen differenzierbar dokumentiert werden, wobei der dritte Identifikator in den ersten Bereich (1.B) des Datenblocks (B) geschrieben wird.

12. Verfahren nach einem der vorangehenden Verfahrensansprüche, bei dem alle Geräte (DG, RO) dieselben Rechenvorschriften zur Berechnung des ersten Wertes (W1) und des zweiten Wertes (W2) anwenden.

13. Computerprogramm mit Programmcode, der für das Ausführen eines Verfahrens nach einem der vorhergehenden Verfahrensansprüche geeignet ist, wenn das Computerprogramm auf einem Computer oder einer Computer-basierten Verarbeitungseinheit eines Gerätes (DG, RO) oder einem Dokumentationsmodul (DM) ausgeführt wird.

14. Dokumentationsmodul (DM) für ein Gerät (DG, RO) für das medizinische Umfeld, umfassend:

    - eine Einleseschnittstelle (I) zum Einlesen eines ersten Identifikators (ID-P, ID-RO) und eines zweiten Identifikators (ID-D);
    - eine elektronische Verarbeitungseinheit (V), die ausgebildet ist, eine Applikation zur Durchführung des Verfahrens nach einem der vorangehenden Verfahrensansprüche auszuführen;
    - eine Schnittstelle zu Speicherstrukturen (MEM, DL) zur Speicherung des beschriebenen Datenblocks (B);
    - einer Schnittstelle zu dem Netzwerk (DL), über das die Geräte (DG, RO) in Datenaustausch stehen.

15. Dokumentationsmodul (DM) gemäß dem unmittelbar vorangehenden Anspruch, wobei das Dokumentationsmodul (DM) mit einer Schnittstelle zu weiteren Dokumentationsmodulen (DM) von anderen Geräten (DG, RO) und/oder Teilnehmern des Dokumentationsverfahrens ausgebildet ist.

16. Gerät (DG, RO) mit einem Dokumentationsmodul (DM), gemäß einem der vorangehenden auf das Dokumentationsmodul (DM) gerichteten Ansprüche.

17. Gerät (DG, RO) nach dem unmittelbar vorangehenden Anspruch, das einen Speicher (MEM) umfasst, der Bestandteil einer verteilten Datenstruktur (DL) ist, die auf mehreren Speichern von unterschiedlichen Geräten (DG, RO) ausgebildet ist und/oder wobei der Speicher (MEM) zur Speicherung von mehreren Ketten von Blöcken umfasst.

18. System zur Dokumentation eines Gerätebetriebs eines Gerätes (DG, RO) im medizinischen Umfeld, wobei der Gerätebetrieb mit einem Verknüpfungsereignis zwischen zwei Verknüpfungspartnern einhergeht, wobei einer der Verknüpfungspartner ein Gebrauchsgegenstand (D) ist, der bei dem Gerätebetrieb verwendet wird, wobei das Gerät (DG, RO) in einem verteilten Geräteverbund betrieben wird und wobei jeweils ein Gerät (DG, RO) ein Dokumentationsmodul (DM) gemäß einem der vorangehenden auf das Dokumentationsmodul (DM) gerichteten Ansprüche umfasst.

19. System nach dem vorangehenden Anspruch, bei dem die Speicher (MEM) der Geräte (DG, RO) eine verteilte Speicherstruktur (DL) bilden.

20. System nach einem der vorangehenden auf das System gerichteten Ansprüche, bei dem ein erster Verknüpfungspartner ein Patient (P) ist, dem ein ihn eineindeutig identifizierender erster Identifikator (ID-P) zugeordnet ist und bei dem ein zweiter Verknüpfungspartner ein medizinischer Gebrauchsgegenstand (D) ist, der mit einer Kennzeichnung (L) gekennzeichnet ist, wobei der Kennzeichnung (L) auf eineindeutige Weise ein zweiter Identifikator (ID-D) zugeordnet ist und bei dem das Verknüpfungsereignis die Verwendung des jeweiligen Gebrauchsgegenstandes (D) für den jeweiligen Patienten (P) bei einer Behandlung an einem bestimmten medizinischen Gerät (DG) repräsentiert.

21. System nach einem der vorangehenden Ansprüche, bei dem ein erster Verknüpfungspartner eine Umkehrosmose-Anlage (RO) ist, wobei die Umkehrosmose-Anlage (RO) mit einer sie eineindeutig identifizierenden Kennzeichnung ausgebildet ist, wobei der Kennzeichnung auf eineindeutige Weise ein erster Identifikator (ID-RO) zugeordnet ist und bei dem ein zweiter Verknüpfungspartner eine Membran (M) ist, die mit einer sie eindeutig identifizierenden Kennzeichnung (L) gekennzeichnet ist, der auf eineindeutige Weise ein zweiter Identifikator (ID-D) zugeordnet ist und bei dem das Verknüpfungsereignis die Verwendung der Membran (M) in der Umkehrosmose-Anlage (RO) repräsentiert.

Fig. 1

EP 3 699 922 A1

Fig. 2

EP 3 699 922 A1

| he1 | B1 | fo1 | he2 | B2 | fo2 | he3 | B3 | fo3 | ... |
|-----|-----|-----|-----|-----|-----|-----|-----|-----|-----|
| f (ID-D1) | g (ID-P1) | | f (ID-D1) | g (ID-P1) | | f (ID-D1) | g (ID-P1) | | ... |
| f (ID-D2) | g (ID-P1) | | f (ID-D2) | g (ID-P1) | | f (ID-D2) | g (ID-P1) | | ... |
| f (ID-D3) | g (ID-P2) | | f (ID-D3) | g (ID-P2) | | ... | ... | | |

Fig. 3

B

f (ID-D)

g (ID-P)

z (D)

he

fo

Fig. 4

Fig. 5

Fig. 6

EP 3 699 922 A1

EP 3 699 922 A1

ID-RO

RV1(ID-RO)
-> W1

ID-P

RV1(ID-P)
-> W1

W1 -> 1.B

START → S1a → S2a → S3a → S4a
S1a → ... → S5 → S6 → ENDE

START → S1b → S2b → S3b → S4b → S5

ID-D

RV2(ID-D)
-> W2

W2 -> 2.B

Fig. 7

**Europäisches Patentamt**
**European Patent Office**
**Office européen des brevets**

# EUROPÄISCHER RECHERCHENBERICHT

Nummer der Anmeldung

EP 19 15 8311

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2018/096121 A1 (GOERINGER STEVEN JOHN [US] ET AL) 5. April 2018 (2018-04-05) * Absätze [0006], [0019], [0025], [0026], [0029], [0030], [0033], [0036], [0037], [0039], [0041], [0042], [0047], [0051], [0052]; Ansprüche 6,13; Abbildung 1 * ----- | 1-21 | INV. G16H40/40 G16H40/60 G06F21/00 H04L29/08 |
| X | US 2017/295157 A1 (CHAVEZ DAVID [US] ET AL) 12. Oktober 2017 (2017-10-12) * Absätze [0036] - [0039], [0047], [0060] - [0062]; Abbildungen 6,7 * ----- | 1 | |
| X | Anonymous: "Distributed-Ledger-Technologie - Wikipedia", , 11. Januar 2019 (2019-01-11), XP055610156, Gefunden im Internet: URL:https://de.wikipedia.org/w/index.php?title=Distributed-Ledger-Technologie&oldid=184609978 [gefunden am 2019-07-31] * das ganze Dokument, insbesondere Einleitung und Abschnitt "Terminologie" * ----- | 1 | RECHERCHIERTE SACHGEBIETE (IPC) G16H H04L G06F |

Der vorliegende Recherchenbericht wurde für alle Patentansprüche erstellt

| Recherchenort | Abschlußdatum der Recherche | Prüfer |
|---|---|---|
| München | 6. August 2019 | Heidrich, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 19 15 8311

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-08-2019

| Im Recherchenbericht angeführtes Patentdokument | Datum der Veröffentlichung | Mitglied(er) der Patentfamilie | Datum der Veröffentlichung |
|---|---|---|---|
| US 2018096121 A1 | 05-04-2018 | KEINE | |
| US 2017295157 A1 | 12-10-2017 | CN 107277810 A | 20-10-2017 |
| | | JP 2017187777 A | 12-10-2017 |
| | | US 2017295157 A1 | 12-10-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- WO 2015024647 A2